# EUROPEAN PATENT APPLICATION

(11) **EP 4 663 663 A1**
(43) Date of publication of application: **17.12.2025**
(21) Application number: 23920828.3
(22) Date of filing: 24.11.2023
(51) Int. Cl.: C07K 19/00, C12N 15/62, C12N 15/867, C12N 5/10, A61K 39/00, A61P 35/00

(54) **USE OF NATURAL PROTEIN TSH AS ANTIGEN-BINDING SITE IN CONSTRUCTION OF CAR-T CELLS TARGETING TSHR**

(30) Priority: 07.02.2023 CN 202310076013
(71) Applicant: Xuzhou Medical University, Xuzhou, Jiangsu 221004 (CN)
(72) Inventor: DU, Hongwei, Xuzhou, Jiangsu 221004 (CN); WANG, Fei, Xuzhou, Jiangsu 221004 (CN); ZUO, Hui, Xuzhou, Jiangsu 221004 (CN); ZHENG, Junnian, Xuzhou, Jiangsu 221004 (CN)
(74) Representative: Valet Patent Services Limited
(86) International application number: PCT/CN2023/134086
(87) International publication number: WO 2024/164637

(57) **Abstract**

The present invention provides a CAR-T cell constructed on the basis of a natural protein TSH. The present invention provides a chimeric antigen receptor constructed on the basis of a TSHα subunit and TSHβ subunit tandem structure, a CAR-T cell, and a use thereof. The TSHαβ-CAR-T cell of the present invention can kill TSHR-positive thyroid cancer cells in a targeted manner, and has no immunogenicity, so that the TSHαβ-CAR-T cell has the prospect of being used in the treatment of thyroid cancer.

## Description

### TECHNICAL FIELD

The present application relates to the field of biomedicine. Specifically, the present application relates to a CAR-T cell constructed based on a natural protein TSH.

### BACKGROUND ART

CAR-T cell immunotherapy has achieved breakthroughs in hematologic malignancies, and several cell therapy drugs has now been used in clinical treatment of patients. However, the treatment of solid tumors faces challenges such as lack of effective antigenic targets and the short survival time of CAR-T cells, which lead to poor therapeutic efficacy. In the above, the immunogenicity of CAR molecules causes that the problem of CAR-T cells being cleared by the patient's immune system occurs in both the hematologic malignancy and solid tumors, significantly reducing the therapeutic efficacy of CAR-T cells and their ability to prevent tumor recurrence. Therefore, it is crucial to find new targets and construct non-immunogenic CAR molecules for improving the therapeutic efficacy of CAR-T cells in solid tumors.

Thyroid cancer is a common malignant tumor of the endocrine system, and its incidence has increased rapidly worldwide in recent years. While traditional therapies such as surgery treatment and radioactive iodine therapy have good therapeutic efficacy for most patients, approximately 10% of patients still die due to tumor metastasis or insensitivity to traditional therapies. Cellular immunotherapy may offer new opportunities for these patients, thus discovering antigenic targets for thyroid cancer and developing CAR-T cell therapies are of important clinical significance. TSHR is primarily expressed on the surface of the thyroid cell and highly expressed in the thyroid cancer cell, and demonstrates potential as a therapeutic target for thyroid-related diseases. CAR-T cells targeting the thyroid-stimulating hormone receptor (TSHR) may effectively kill thyroid tumor cells.

A recent study found that CAR-T cells constructed based on the scFv sequence of TSHR monoclonal antibody may effectively kill TSHR-positive thyroid cancer cells, proving the feasibility of treating thyroid cancer with TSHR used as a target. However, there are still problems that the immunogenicity of CAR in CAR-T cell therapy causes the inability of CAR-T cells to persist in the body.

Therefore, there is a need in the art to develop a non-immunogenic CAR immune cell targeting TSHR.

### SUMMARY

The purpose of the present application is to provide a non-immunogenic CAR immune cell targeting TSHR.

In a first aspect of the present application, a chimeric antigen receptor (CAR) is provided, where the CAR contains an extracellular antigen-binding domain targeting TSHR, and the extracellular antigen-binding domain includes a structure as shown by the following formula (I):

Tα-L-Tβ (I),

in the above,
each "-" independently indicates a linker peptide or a peptide bond;
Tα is a TSH protein α subunit, or a fragment thereof;
Tβ is a TSH protein β subunit, or a fragment thereof; and
L is nothing (absent) or a linker peptide.

In another preferred embodiment, the accession number of the TSH protein α subunit is NM_001303177.1.

In another preferred embodiment, the accession number of the TSH protein β subunit is NM_000549.5.

In another preferred embodiment, the fragment of the TSH protein α subunit is a fragment obtained by truncating 0-10 amino acids from the N-terminus and/or 0-10 amino acids from the C-terminus of the TSH protein α subunit as shown in SEQ ID NO: 2.

In another preferred embodiment, the fragment of the TSH protein α subunit is a fragment obtained by truncating 1-10 amino acids, preferably 1-6 amino acids, and more preferably 1-2 amino acids, from the C-terminus of the TSH protein α subunit as shown in SEQ ID NO: 2.

In another preferred embodiment, the amino acid sequence of the Tα is as shown in SEQ ID NO: 2 or SEQ ID NO: 19, or has a sequence identity of ≥ 85% (preferably ≥ 90%, more preferably ≥ 95%, for example ≥ 96%, ≥ 97%, ≥ 98% or ≥ 99%) to and has the same or substantially the same binding function as the sequence as shown in SEQ ID NO: 2 or SEQ ID NO: 19.

In another preferred embodiment, the fragment of the TSH protein β subunit is a fragment obtained by truncating 0-10 amino acids from the N-terminus and/or 0-20 amino acids from the C-terminus of the TSH protein β subunit as shown in SEQ ID NO: 4.

In another preferred embodiment, the fragment of the TSH protein β subunit is a fragment obtained by truncating 1-20 amino acids, and preferably 1-16, 1-14 or 1-12 amino acids, from the C-terminus of the TSH protein β subunit as shown in SEQ ID NO: 4.

In another preferred embodiment, the amino acid sequence of the Tβ is as shown in SEQ ID NO: 4, 20, 21 or 22, or has a sequence identity of ≥ 85% (preferably ≥ 90%, more preferably ≥ 95%, for example ≥ 96%, ≥ 97%, ≥ 98% or ≥ 99%) to and has the same or substantially the same binding function as the sequence as shown in SEQ ID NO: 4, 20, 21 or 22.

In another preferred embodiment, the L is a flexible linker peptide.

In another preferred embodiment, the sequence of the L is (G4S)n, where n is an integer selected from 1-6, and preferably n is 2, 3 or 4.

In another preferred embodiment, the sequence of the L is as shown in SEQ ID NO: 32.

In another preferred embodiment, the extracellular antigen-binding domain of the CAR further includes a second extracellular domain targeting an additional target.

In another preferred embodiment, the additional target is a tumor-specific target, and preferably a thyroid cancer-specific target.

In another preferred embodiment, the structure of the CAR is as shown by the following formula II:

S-EB-F-H-TM-C-CD3ζ (II)

in the formula,
each " -" is independently a linker peptide or a peptide bond;
S is nothing (absent) or a signal peptide sequence;
EB is the extracellular antigen-binding domain;
H is nothing (absent) or a hinge region;
TM is the transmembrane domain;
C is nothing (absent) or intracellular costimulatory domain;
CD3ζ is an intracellular domain coding sequence derived from CD3ζ; and
F is nothing (absent) or a labeled protein.

In another preferred embodiment, the labeled protein F can be detected directly or indirectly.

In another preferred embodiment, the CAR can be detected by an antibody that specifically targets the labeled protein F.

In another preferred embodiment, the amino acid sequence of the labeled protein F is as shown in SEQ ID NO: 31.

In another preferred embodiment, the S is a signal peptide of a protein selected from the following group: CD8, CD28, GM-CSF, CD4, CD137 and a combination thereof.

In another preferred embodiment, the H is the hinge region of a protein selected from the following group: CD8, CD28, CD137 and a combination thereof.

In another preferred embodiment, the H is a hinge region derived from CD8α.

In another preferred embodiment, the TM is a transmembrane region of a protein selected from the following group: CD28, CD3 epsilon, CD45, CD4, CD5, CD8, CD9, CD16, CD22, CD33, CD37, CD64, CD80, CD86, CD134, CD137, CD154 and a combination thereof.

In another preferred embodiment, the TM is a transmembrane region derived from CD8α.

In another preferred embodiment, the amino acid sequence of the H-TM is as shown in SEQ ID NO: 6.

In another preferred embodiment, the C is an intracellular costimulatory domain of a protein selected from the following group: OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2 and a combination thereof.

In another preferred embodiment, the C is an intracellular costimulatory domain derived from 4-1BB or CD28.

In another preferred embodiment, the amino acid sequence of the C is as shown in SEQ ID NO: 8 or 10.

In another preferred embodiment, the amino acid sequence of the intracellular domain coding sequence of CD3ζ is as shown in SEQ ID NO: 12.

In another preferred embodiment, the amino acid sequence of the chimeric antigen receptor CAR is as shown in SEQ ID NO: 14, 16, 24, 26, 28 or 30, or has a sequence identity of ≥ 85% (preferably ≥ 90%, more preferably ≥ 95%, for example ≥ 96 %, ≥ 97%, ≥ 98% or ≥ 99%) to the sequence as shown in SEQ ID NO: 14, 16, 24, 26, 28 or 30.

In a second aspect of the present application, a nucleic acid molecule is provided, where the nucleic acid molecule encodes the chimeric antigen receptor according to the first aspect of the present application.

In another preferred embodiment, the nucleic acid molecule has the nucleotide sequence as shown in SEQ ID NO: 13, 15, 23, 25, 27 or 29.

In the third aspect of the present application, a vector is provided, where the vector contains the nucleic acid molecule according to the second aspect of the present application.

In another preferred embodiment, the vector is selected from the following group: DNA, RNA, plasmid, lentiviral vector, adenoviral vector, retroviral vector, transposon and a combination thereof.

In another preferred embodiment, the vector is the retroviral vector, and preferably a SFG retroviral vector.

In another preferred embodiment, the vector further includes that selected from the following group: a promoter, a transcriptional enhancer element WPRE, a long terminal repeat sequence LTR, and the like.

In another preferred embodiment, the vector contains the nucleotide sequence as shown in SEQ ID NO: 13, 15, 23, 25, 27 or 29.

In the fourth aspect of the present application, a host cell is provided, where the host cell contains the vector according to the third aspect of the present application, or is integrated with an exogenous nucleic acid molecule according to the second aspect of the present application in a chromosome, or expresses the CAR according to the first aspect of the present application.

In the fifth aspect of the present application, an engineered immune cell is provided, where the immune cell contains the vector according to the third aspect of the present application, or is integrated with an exogenous nucleic acid molecule according to the second aspect of the present application in a chromosome, or expresses the CAR according to the first aspect of the present application.

In another preferred embodiment, the engineered immune cell is selected from the following group: a T cell, a NK cell, a NKT cell, a macrophage and a combination thereof.

In another preferred embodiment, the engineered immune cell is a chimeric antigen receptor T cell (CAR-T cell) or a chimeric antigen receptor NK cell (CAR-NK cell).

In another preferred embodiment, the engineered immune cell is a CAR-T cell.

In the sixth aspect of the present application, a method for preparing the engineered immune cell according to the fifth aspect of the present application is provided, including following steps: transducing the nucleic acid molecule according to the second aspect of the present application or the vector according to the third aspect of the present application into an immune cell, to obtain the engineered immune cell.

In another preferred embodiment, the method further includes the step of performing function and effectiveness testing on the obtained engineered immune cell.

In the seventh aspect of the present application, a pharmaceutical composition is provided, where the pharmaceutical composition contains the CAR according to the first aspect of the present application, the nucleic acid molecule according to the second aspect of the present application, the vector according to the third aspect of the present application, the host cell according to the fourth aspect of the present application, and/or the engineered immune cell according to the fifth aspect of the present application, and a pharmaceutically acceptable carrier, diluent or excipient.

In another preferred embodiment, the formulation is a liquid formulation.

In another preferred embodiment, the formulation is in the form of an injection.

In another preferred embodiment, the concentration of the engineered immune cell in the formulation is 1×10³ -1×10⁸ cells/ml, and preferably 1×10⁴ -1×10⁷ cells/ml.

In the eighth aspect of the present application, a use of the CAR according to the first aspect of the present application, the nucleic acid molecule according to the second aspect of the present application, the vector according to the third aspect of the present application, or the host cell according to the fourth aspect of the present application, and/or the engineered immune cell according to the fifth aspect of the present application in preparation of a drug or a formulation for preventing and/or treating a disease associated with high TSHR expression is provided.

In another preferred embodiment, the disease associated with high TSHR expression includes tumor, Graves' disease, or a combination thereof.

In another preferred embodiment, the disease associated with high TSHR expression is cancer or tumor, such as thyroid tumor.

In the ninth aspect of the present application, the engineered immune cell according to the fifth aspect of the present application or the pharmaceutical composition according to the seventh aspect of the present application for use in preventing and/or treating a disease associated with high TSHR expression is provided.

In another preferred embodiment, the disease associated with high TSHR expression includes tumor, Graves' disease, or a combination thereof.

In another preferred embodiment, the disease associated with high TSHR expression is cancer or tumor, such as thyroid cancer.

In the tenth aspect of the present application, a method for treating a disease associated with high TSHR expression is provided, including administering an effective amount of the engineered immune cell according to the fifth aspect of the present application or the pharmaceutical composition according to the seventh aspect of the present application to a subject in need of treatment.

In another preferred embodiment, the disease associated with high TSHR expression includes cancer or tumor, Graves' disease, or a combination thereof.

In another preferred embodiment, the disease is cancer or tumor, such as thyroid cancer.

In another preferred embodiment, the engineered immune cell or CAR immune cell contained in the pharmaceutical composition is derived from a cell of the subject (autologous cell).

In another preferred embodiment, the engineered immune cell or CAR immune cell contained in the pharmaceutical composition is a cell derived from a healthy individual (allogeneic cell).

In another preferred embodiment, the method can be used in combination with other treatment methods.

In another preferred embodiment, the other treatment methods include chemotherapy, radiotherapy, targeted therapy and the like.

It should be understood that within the scope of the present application, the above-mentioned technical features of the present application and the technical features described in detail below (such as in the embodiments) may be combined with each other to form new or preferred technical solutions. Due to space limitations, they will not be enumerated here one by one.

### BRIEF DESCRIPTION OF DRAWINGS

The following drawings are used to illustrate specific embodiments of the present application and are not used to limit the scope of the present application defined by the claims.

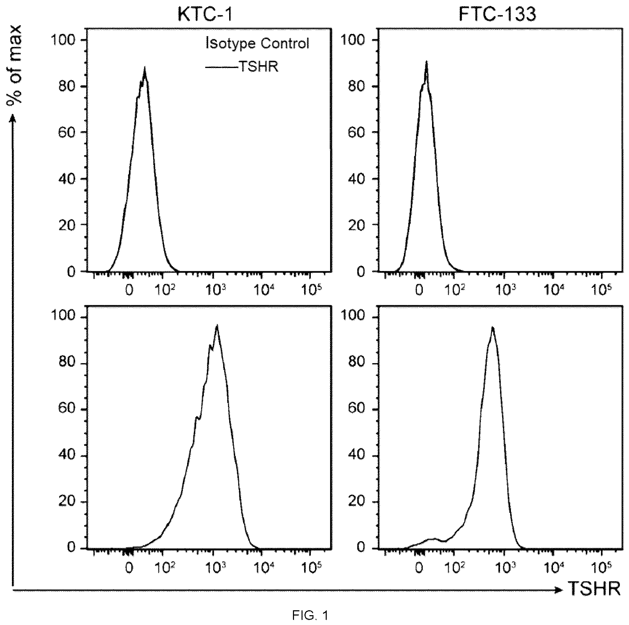

FIG. 1 shows the construction results of TSHR-overexpressing thyroid cancer cell strains.

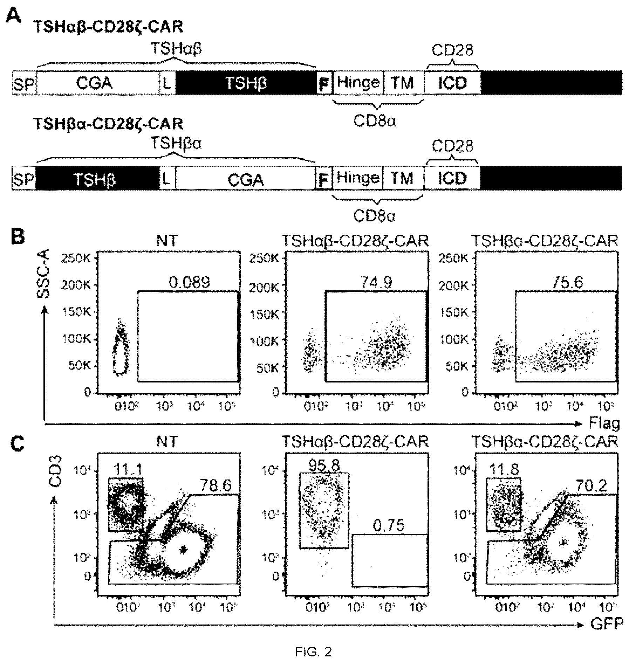

FIG. 2 shows the schematic structure views of TSHαβ-CAR and TSHβα-CAR and their killing effects on TSHR-overexpressing thyroid cancer cells KTC-1. In the above, (A) shows the schematic structure views of TSHαβ-CD28ζ-CAR and TSHβα-CD28ζ-CAR; SP represents the signal peptide sequence; L represents the (G4S)₃ linker peptide sequence; F represents the Flag tag sequence; Hinge and TM represent the hinge region and transmembrane region domain of CD8α, respectively; ICD represents the intracellular costimulatory domain of CD28; and CD3ζ represents the intracellular domain of CD3ζ. (B) shows expression of TSHaβ-CAR and TSHβα-CAR on T cells detected by the flow cytometry after staining with an anti-Flag antibody. (C) shows the ratio of T cells (CD3⁺) to residual tumor cells (GFP⁺) detected by the flow cytometry after coculturing the NT, TSHαβ-CAR-T and TSHβα-CAR-T cells respectively with the TSHR-overexpressing thyroid cancer cell strain KTC-1 at an effector-to-target ratio of 1:5 for 5 days.

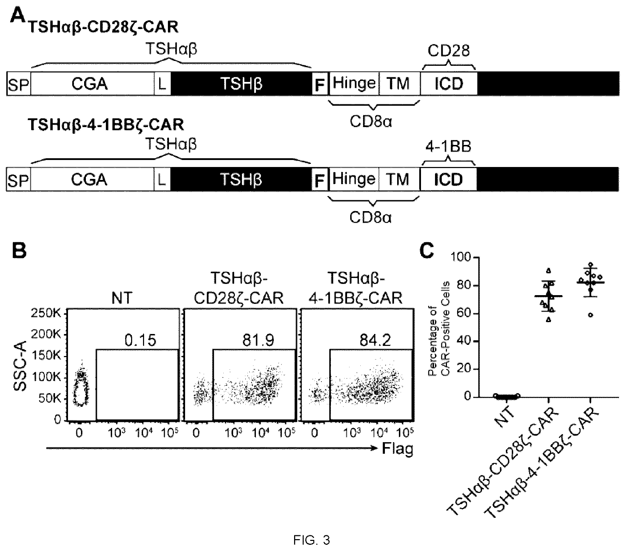

FIG. 3 shows the schematic structure views of the TSHαβ-CAR and T cell infection efficiency. In the above, (A) shows the schematic structure views of TSHαβ-CD28ζ-CAR and TSHβα-4-1BBζ-CAR; SP represents the signal peptide sequence; L represents the (G4S)₃ linker peptide sequence; F represents the Flag tag sequence; Hinge and TM represent the hinge region and transmembrane region domain of CD8α, respectively; ICD represents the intracellular costimulatory domain of CD28 or 4-1BB; and CD3ζ represents the intracellular domain of CD3ζ. (B) shows expression of TSHαβ-CAR on the T cells after staining with an anti-Flag antibody. (C) shows infection rate statistical results of TSHαβ-CAR-expressing viral infection in T cells from different volunteers.

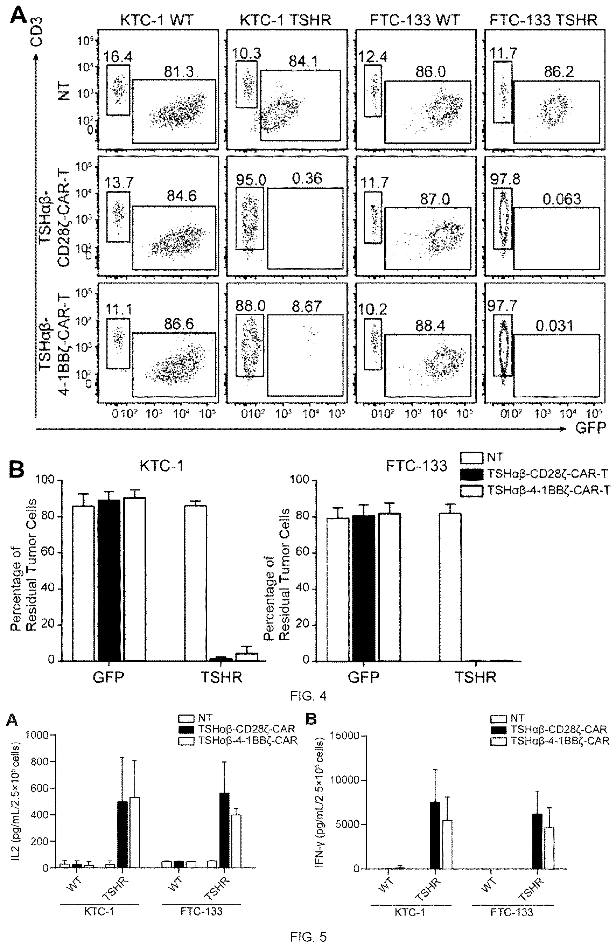

FIG. 4 shows that the TSHαβ-CAR-T cell specifically kills the TSHR-overexpressing thyroid cancer cell strain. In the above, (A) shows the ratio of T cells (CD3⁺) to residual tumor cells (GFP⁺) detected by the flow cytometry after coculturing the NT and TSHαβ-CAR-T cells respectively with a control group and a TSHR-overexpressing group of the thyroid cancer cell strain at an effector-to-target ratio of 1:5 for 5 days. (B) shows the statistical results of the proportions of residual tumor cells in different groups after the coculture experiment is ended.
FIG. 5 shows that the TSHαβ-CAR-T cell releases large amounts of cytokines IFN-γ and IL-2 when killing TSHR-positive tumor cell. In the above, NT and TSHαβ-CAR-T cells are cocultured with the control group and the TSHR-overexpressing group of the thyroid cancer cell strain respectively, at an effector-target ratio of 1:5 for 24 h, and then the coculture supernatant is collected and the secretion amounts of IFN-γ and IL-2 in the supernatant are detected by ELISA.

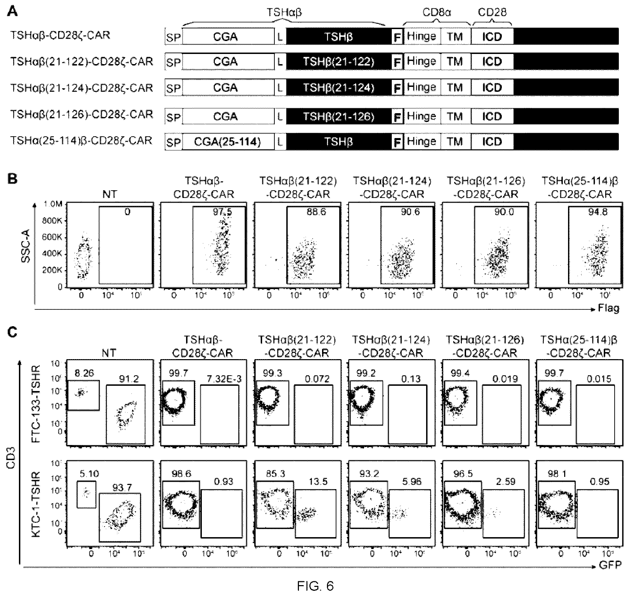

FIG. 6 shows the schematic structure views of various truncated mutant TSHαβ-CARs and their killing effects on TSHR-overexpressing thyroid cancer cells FTC-133 and KTC-1. In the above, (A) shows schematic structure views of the TSHαβ-CAR truncated mutants, where SP represents the signal peptide sequence; F represents the Flag tag sequence; Hinge and TM represent the hinge region and transmembrane region domain of CD8, respectively; ICD represents the intracellular costimulatory domain of CD28; CD3ζ represents the intracellular domain of CD3ζ; and the numbers in parentheses indicate the corresponding TSHα or TSHβ amino acid segments. (B) shows expression of the various truncated mutant TSHαβ-CARs on the T cells detected by the flow cytometry after staining with anti-Flag antibodies. (C) shows the ratios of T cells (CD3⁺) to residual tumor cells (GFP⁺) detected by the flow cytometry after coculturing the T cells expressing the truncated mutant TSHαβ-CARs respectively with TSHR-overexpressing thyroid cancer cell strains FTC-133 and KTC-1 at an effector-to-target ratio of 1:5 for 5 days.

### DETAILED DESCRIPTION OF EMBODIMENTS

After extensive and in-depth researches, the inventors have developed, for the first time, a CAR-T cell based on the natural protein TSH. The present application constructs a non-immunogenic second-generation CAR-T (TSHαβ-CAR-T) of a specific structure based on TSH, the natural ligand of the TSHR, by utilizing the TSH α subunit and β subunit. In-vitro experiments have demonstrated that the TSHαβ-CAR-T cell of the present application can targetedly kill TSHR-positive thyroid cancer cells. Furthermore, truncated mutation of the TSHα and TSHβ (e.g., the truncated mutation of C-terminus) does not affect the recognition and binding of the antigen-binding domain to TSHR; and the CAR-T cells containing the truncated mutants are still able to recognize and kill tumor cells. The present application is completed based on this.

### Terms

In order to make the present application easier to understand, certain technical and scientific terms are specifically defined below. Unless otherwise clearly defined herein, all other technical and scientific terms used herein have the meanings generally understood by those of ordinary skill in the art to which the present application belongs. Before describing the present application, it should be understood that the present application is not limited to the specific methods and experimental conditions described, because such methods and conditions may be changed. It should also be understood that the terms used herein are intended only to describe specific embodiments and are not intended to be restrictive, and the scope of the present application will be limited only by the appended claims.

Unless otherwise defined, all technical and scientific terms used herein have the same meaning as commonly understood by one of ordinary skill in the art to which the present application belongs. As used herein, when used in a specific listed numeral value, the term "approximately (about)" means that the value may vary by no more than 1% from the listed value. For example, as used herein, the expression "approximately 100" includes 99 and 101 as well as all values therebetween (e.g., 99.1, 99.2, 99.3, 99.4, etc.).

As used herein, the terms "optional" or "optionally" mean that the subsequently described event or circumstance may occur but not necessarily occur.

As used herein, the terms "containing" or "including (comprising)" may be open-ended, semi-closed-ended, or closed-ended. In other words, the terms also include "consisting essentially of" or "consisting of".

The term "about" may refer to a value or composition that is within an acceptable error range for the particular value or composition as determined by one of ordinary skill in the art, which will depend in part on how the value or composition is measured or determined.

The term "administering" refers to the physical introduction of the product of the present application into a subject by using any one of a variety of methods and delivery systems known to those skilled in the art, including intravenous, intramuscular, subcutaneous, intraperitoneal, spinal or other parenteral routes of administration, such as by injection or infusion.

"Transduction", "transfection" and "transformation" refer to the process of transferring exogenous polynucleotides into host cells, transcribing and translating to produce polypeptide products, which includes using plasmid molecules to introduce exogenous polynucleotides into host cells (for example, *Escherichia coli*).

"Gene expression" or "expression" refers to the process of producing the RNA or protein product of a gene through gene transcription, translation, and post-translational modification.

"Polynucleotide" refers to a polymerization form of nucleotides of any length, including deoxynucleotides (DNA), ribonucleotides (RNA), and hybrid sequences and analogs thereof. A polynucleotide may include modified nucleotides, such as methylated or capped nucleotides or nucleotide analogs. As used herein, the term polynucleotide refers to single-stranded and double-stranded molecules which are interchangeable. Unless otherwise indicated, the polynucleotide in any embodiment described herein includes a double-stranded form and two complementary single strands that are known or predicted to form a double-stranded form.

Conservative amino acid substitutions are known in the art. In some embodiments, potential substituting amino acids are within one or more of the following groups: glycine, alanine; and valine, isoleucine, leucine, and proline; aspartic acid, glutamic acid; asparagine, glutamine; serine, threonine, lysine, arginine, and histidine; and/or phenylalanine, tryptophan, and tyrosine; and methionine, and cysteine. In addition, the present application also provides non-conservative amino acid substitutions that allow substitution of amino acids from different groups.

### TSHR and TSH

TSHR is a glycoprotein receptor expressed on the surface of cell membrane, mainly expressed in thyroid cells. It plays a crucial regulatory role in physiological processes such as thyroid growth, differentiation, and thyroid hormone secretion. In pathological conditions, TSHR is a potential therapeutic target for Graves' disease. Meanwhile, TSHR is constitutively expressed in the vast majority of differentiated thyroid cancer cells. In-vivo experiments in mice have shown that the metastasis of differentiated thyroid cancer cells requires TSHR-mediated growth signals. TSHR positivity has also been found in primary and metastatic lesions of thyroid cancer in clinical samples. These results suggest that TSHR may be a potential therapeutic target for differentiated thyroid cancer.

As used herein, the terms "TSH protein α subunit" and " TSHα" both refer to the α subunit of the thyroid-stimulating hormone (TSH) protein. The terms "TSH protein β subunit" and "TSHβ" both refer to the β subunit of the thyroid-stimulating hormone (TSH) protein. Thyroid-stimulating hormone (TSH) is a hormone secreted by the adenohypophysis. TSH is a glycoprotein composed of two non-covalently linked subunits. The α subunit thereof is common to all hormones of this type, while the β subunit is unique.

### Chimeric Antigen Receptor (CAR)

Chimeric immune antigen receptor (Chimeric antigen receptor, CAR) consists of an extracellular antigen recognition region, a transmembrane region, and an intracellular costimulatory signaling region.

The design of CAR has evolved through the following processes: the first-generation CAR contains only one intracellular signaling component, CD3ζ or FcγRI molecule, and due to the only one intracellular activation domain, it may only trigger transient T cell proliferation and less cytokine secretion, but cannot provide prolonged T cell proliferation signaling and sustained in-vivo anti-tumor effect, resulting in not good clinical efficacy. For the second-generation CAR, one costimulatory molecule, such as CD28, 4-1BB, OX40, and ICOS, is introduced based on the existing structure. Compared with the first-generation CAR, the function is greatly improved, and the persistence of CAR-T cells and their ability to kill tumor cells are further enhanced. New immune costimulatory molecules, such as CD27 and CD134, are incorporated in tandem based on the second-generation CAR, to develop into the third-generation and fourth-generation CARs.

The extracellular segment of CAR may recognize a specific antigen and then transduce the signal through the intracellular domain, causing cell activation and proliferation, cytolytic toxicity, and cytokine secretion, thereby eliminating the target cells. First, the patient's autologous cells (or allogeneic donors) are isolated, activated, and genetically modified to produce CAR-containing immune cells, which are then injected into the body of the same patient. This approach has a very low probability of having graft-versus-host disease, as the antigen is recognized by immune cells in a non-MHC-restricted manner.

CAR-immune cell therapy has achieved a very high clinical response rate in the treatment of hematological malignancies, and such a high response rate cannot be achieved by any previous treatment means, and has triggered a wave of clinical research around the world.

Specifically, the chimeric antigen receptor (CAR) of the present application includes an extracellular domain, a transmembrane domain, and an intracellular domain.

The extracellular domain includes a specific antigen-binding domain. The extracellular domain is a natural sequence or a derivative thereof for mediating the specific binding of ligand-receptor.

The CAR-T cell of the present application is constructed by means of expressing two subunits of TSH in tandem, where the TSH is used as an antigen-binding site, instead of the scFv sequence of the TSHR monoclonal antibody. The present application successfully constructs a CAR-T cell expressing CAR with tandem TSHα and β subunits, and confirms through in-vitro experiments that it can specifically target and kill TSHR-positive thyroid cancer cells. In traditional CAR molecules, the scFv sequence that recognizes and binds to antigens is generally the variable region sequence of a murine monoclonal antibody, which is immunogenic in the human body, and after infused into the patient's body, the CAR-T cell is easy to be cleared by the patient's immune system, resulting in poor therapeutic effect or tumor recurrence. However, the TSH, as a natural protein present in the human body, does not have any immunogenicity, can effectively avoid the immunogenicity problem of CAR molecule, and can better promote the survival of the CAR-T cell in the body, play a long-term monitoring role, and prevent tumor recurrence.

In the present application, the antigen-binding domain of the chimeric antigen receptor is composed of a α subunit (or a fragment thereof) and a β subunit (or a fragment thereof) of a TSH protein connected in tandem, and includes a structure as shown by the following formula (I):

Tα-L-Tβ (I)

In the above, each "-" is independently a linker peptide or a peptide bond;
Tα is the TSH protein α subunit, or a fragment thereof;
Tβ is the TSH protein β subunit, or a fragment thereof; and
L is nothing or a linker peptide.

In one embodiment, TSHα and/or TSHβ in the antigen-binding domain may be subjected to truncation mutation, for example, truncating 1-20 amino acids from the C-terminus. Such truncation mutation does not or substantially does not affect the recognition and binding of TSHR by the antigen-binding domain. The CAR-T cell constructed based on the truncated mutant can still recognize and kill tumor cells.

In some embodiments, the TSHα fragment may be obtained by truncating 0-10 amino acids from the N-terminus and/or truncating 0-10 amino acids from the C-terminus of the full-length sequence of TSHα as shown in SEQ ID NO: 2. For example, 0, 1, 2, 3, 4, 5, 6, 7, 8, 9, or 10 amino acids may be truncated from the N-terminus; and/or 0, 1, 2, 3, 4, 5, 6, 7, 8, 9, or 10 amino acids may be truncated from the C-terminus.

In some embodiments, the TSHβ fragment may be obtained by truncating 0-10 amino acids from the N-terminus and/or 0-20 amino acids from the C-terminus of the full-length sequence of TSHβ as shown in SEQ ID NO: 4. For example, 0, 1, 2, 3, 4, 5, 6, 7, 8, 9, or 10 amino acids may be truncated from the N-terminus; and/or 0, 1, 2, 3, 4, 5, 6, 7, 8, 9, 10, 11, 12, 13, 14, 15, 16, 17, 18, 19 or 20 amino acids may be truncated from the N-terminus.

The intracellular domain includes a costimulatory signaling region and a ζ chain portion. The costimulatory signaling region refers to a portion of the intracellular domain that includes costimulatory molecules. the costimulatory molecules are cell surface molecules required for lymphocytes to effectively respond to antigens, rather than antigen receptors or their ligands.

A linker may be incorporated between the extracellular domain and the transmembrane domain of CAR, or between the cytoplasmic domain and the transmembrane domain of CAR. The linker may be any oligopeptide or polypeptide that plays the role of connecting the transmembrane domain to the extracellular domain or cytoplasmic domain of a polypeptide chain. The linker may include 0-300 amino acids, preferably 2 to 100 amino acids, and most preferably 3 to 50 amino acids.

When expressed in the T cell, the CAR of the present application can carry out antigen recognition based on antigen-binding specificity. When binding to its associated antigen, it affects tumor cells, causing tumor cells to not grow, be prompted to die or be affected in other way, and causes the patient's tumor load to shrink or be eliminated. The antigen-binding domain is preferably fused with intracellular domains from one or more of costimulatory molecules and ζ chains. Preferably, the antigen-binding domain is fused with the intracellular domain of the combination of the CD28 or 4-1BB costimulatory domain and CD3ζ signaling domain.

In the present application, the subunit element constituting the extracellular antigen-binding domain of the present application further includes a sequence-based conservative variant, which refers to the polypeptide formed by replacing at most 10, preferably at most 8, more preferably at most 5, and most preferably at most 3 amino acids by amino acids with similar or analogous properties, compared with the amino acid sequence as shown in SEQ ID NO: 2 or SEQ ID NO: 4.

In the present application, the number of added, deleted, modified and/or substituted amino acids is preferably no more than 40%, more preferably no more than 35%, more preferably 1-33%, more preferably 5-30%, more preferably 10-25%, and more preferably 15-20% of the total number of amino acids in the original amino acid sequence.

In the present application, the number of the added, deleted, modified and/or substituted amino acids is generally 1, 2, 3, 4 or 5, preferably 1-3, more preferably 1-2, and most preferably 1.

For the hinge region and the transmembrane region (transmembrane domain), CAR may be designed to include a transmembrane domain fused to the extracellular domain of CAR. In one embodiment, a transmembrane domain naturally associated with one of the domains in CAR is used. In some examples, the transmembrane domain may be selected or modified by amino acid substitution to avoid the binding of such domain to the transmembrane domain of the same or different surface membrane proteins, thereby minimizing interaction with other members of the receptor complex.

### Chimeric antigen receptor immune cell (CAR-immune cell)

In the present application, a chimeric antigen receptor immune cell is provided, containing the chimeric antigen receptor according to the first aspect of the present application.

The chimeric antigen receptor immune cell of the present application may be a CAR-T cell, or may be a CAR-NK cell, or a CAR-macrophage. Preferably, the chimeric antigen receptor immune cell of the present application is a CAR-T cell.

As used herein, the terms "CAR-T cell", "CAR-T", and "CAR-T cell of the present application" all refer to the CAR-T cell according to the fifth aspect of the present application.

The CAR-T cell has the following advantages compared with other T-cell-based therapy methods: (1) the acting process of the CAR-T cell is not restricted by MHC; (2) since many tumor cells express the same tumor markers, once the CAR gene construction for some tumor marker is completed, it can be widely used; (3) CAR may utilize both the tumor protein marker and the glycolipid non-protein marker, expanding the target range of the tumor marker; (4) the use of the patient's autologous cell reduces the risk of rejection reaction; and (5) the CAR-T cell has immune memory function and can survive in the body for a long time.

As used herein, the terms "CAR-NK cell", "CAR-NK" and "CAR-NK cell of the present application" all refer to the CAR-NK cell according to the fifth aspect of the present application. The CAR-NK cell of the present application may be used for tumors with high TSHR expression.

Natural killer (NK) cells are a major type of immune effector cells that protect the body from viral infection and tumor cell invasion through non-antigen-specific pathways. Engineered (genetically modified) NK cells may obtain new functions, including the ability to specifically recognize tumor antigens and possess enhanced anti-tumor cytotoxicity effect.

Compared with the CAR-T cell, the CAR-NK cell has the following advantages of for example: (1) directly killing tumor cells by releasing perforin and granzymes, but having no killing effect on normal cells in the body; (2) releasing a very small amount of cytokines, thereby reducing the risk of cytokine storm; and (3) being extremely easy to expand in vitro and develop into "ready-made" products. Other than that, it is similar to CAR-T cell therapy.

### Vector

A nucleic acid sequence encoding a desired molecule may be obtained using recombinant methods known in the art, for example, by screening libraries from cells expressing the gene, by obtaining the gene from a vector known to include the gene, or by using a standard technology to isolate the gene directly from cells and tissues that contain the gene. Optionally, the gene of interest may be produced synthetically.

The present application also provides a vector containing the nucleic acid molecule of the present application. The vector derived from retroviruses such as lentiviruses is a suitable tool for realizing long-term transgenosis because they allow long-term, stable integration of the transgene and the proliferation thereof in its daughter cells. The lentiviral vector has advantages over vectors derived from oncogenic retroviruses such as murine leukemia viruses, because they can transduce non-proliferating cells such as hepatocytes. They also have the advantage of low immunogenicity.

In short, an expression kit or nucleic acid sequence of the present application is typically operably linked to a promoter and incorporated into an expression vector. The vector is suitable for replicating and integrating eukaryotic cells. Typical cloning vectors include transcriptional and translational terminators, initial sequences and promoters that can be used to regulate the expression of the desired nucleic acid sequence.

The expression constructs of the present application may also be used in nucleic acid immunization and gene therapy using standard gene delivery solutions. Methods for gene delivery are known in the art; see, e.g., U.S. Patent Nos. 5,399,346, 5,580,859, and 5,589,466, which are incorporated herein in their entirety by reference. In another embodiment, the present application provides a gene therapy vector.

The nucleic acid may be cloned into many types of vectors. For example, the nucleic acid may be cloned into vectors including, but not limited to, plasmids, phagemids, phage derivatives, animal viruses, and cosmids. Particular vectors of interest include expression vectors, replication vectors, probe generation vectors, and sequencing vectors.

Further, expression vectors may be provided to cells in the form of viral vectors. Viral vector techniques are well known in the art and are described, for example, by Sambrook et al. (2001, Molecular Cloning: A Laboratory Manual, Cold Spring Harbor Laboratory, New York) and in other manuals of virology and molecular biology. Viruses that may be used as vectors include, but not limited to, retroviruses, adenoviruses, adeno-associated viruses, herpesviruses, and lentiviruses. Typically, suitable vectors contain an origin of replication, a promoter sequence, convenient restriction enzyme sites, and one or more selectable markers which are functional in at least one organism (e.g., WO01/96584; WO01/29058; and U.S. Patent No. 6,326,193).

A number of virus-based systems have been developed for transferring of genes into mammalian cells. For example, retroviruses provide a convenient platform for gene delivery systems. The selected gene may be inserted into a vector and packaged into retroviral particles by using techniques known in the art. The recombinant virus may then be isolated and delivered to subject cells in vivo or ex vivo. Many retroviral systems are known in the art. In some embodiments, adenoviral vectors are used. Many adenoviral vectors are known in the art. In one embodiment, lentiviral vectors are used.

Additional promoter elements, such as enhancers, may regulate the frequency at the start of transcription. Typically, these are located in a region of 30-110 bp upstream of the start site, although it has recently been shown that many promoters also contain functional elements downstream of the start site. The spacing between promoter elements is often flexible, so that the promoter function is maintained when elements are inverted or moved relative to one another. In the thymidine kinase (tk) promoter, the spacing between promoter elements may be increased by 50 bp before activity begins to decline. Depending on the promoter, individual elements demonstrate cooperative or independent action to initiate transcription.

An example of a suitable promoter is the immediate early cytomegalovirus (CMV) promoter sequence. The promoter sequence is a strong constitutive promoter sequence capable of driving high-level expression of any polynucleotide sequence operably linked to it. Another example of a suitable promoter is elongation growth factor-Iα (EF-1a). However, other constitutive promoter sequences may also be used, including but not limited to the simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus immediate-early promoter, Rous sarcoma virus promoter, and human gene promoters such as but not limited to the actin promoter, myosin promoter, heme promoter and creatine kinase promoter. Further, the present application should not be limited to the use of constitutive promoters. Inducible promoters are also contemplated as a part of the present application. The use of an inducible promoter provides a molecular switch that can turn on the expression of a polynucleotide sequence operably linked to the inducible promoter when such expression is desired or turn off the expression when the expression is not desired. Examples of inducible promoters include, but not limited to, the metallothionein promoter, glucocorticoid promoter, progesterone promoter, and tetracycline promoter.

To evaluate the expression of a CAR polypeptide or portion thereof, the expression vector introduced into cells may also contain either or both of a selectable marker gene and a reporter gene to facilitate the search for identification and selection of expressing cells in the transfected or infected cell population through the viral vector. In other aspects, the selectable marker may be carried on a single section of DNA and used in a co-transfection procedure. Both the selectable marker and the reporter gene may be flanked by appropriate regulatory sequences to enable expression in the host cell. Useful selectable markers include, for example, antibiotic resistance genes such as neo and the like.

A reporter gene is used to identify potentially transfected cells and to evaluate the functionality of regulatory sequences. Typically, a reporter gene is a gene that is not present in the recipient organism or tissue or expressed by the recipient organism or tissue and encodes a polypeptide whose expression is clearly indicated by some readily detectable properties such as enzymatic activity. After the DNA has been introduced into the recipient cell, the expression of the reporter gene is measured at an appropriate time. Suitable reporter genes may include genes coding luciferase, β-galactosidase, chloramphenicol acetyltransferase, secreted alkaline phosphatase, or green fluorescent protein (e.g., Ui-Tei et al., 2000 FEBS Letters 479: 79-82). In one embodiment of the present application, the reporter gene is a gene coding mKate2 red fluorescent protein. Suitable expression systems are well known and may be prepared using known techniques or obtained commercially. Typically, constructs with a minimum of 5 flanking regions showing the highest levels of reporter gene expression are identified as promoters. Such promoter regions may be linked to a reporter gene and used to evaluate the ability of an agent to regulate promoter-driven transcription.

Methods for introducing genes into cells and expressing genes into cells are known in the art. In terms of the content of an expression vector, the vector may be easily introduced by any method known in the art into a host cell, e.g., mammalian, bacterial, yeast or insect cells. For example, an expression vector may be transferred into a host cell by physical, chemical or biological means.

Physical methods for introducing polynucleotides into host cells include calcium phosphate precipitation, lipofection, particle bombardment, microinjection, electroporation, and the like. Methods for producing cells including a vector and/or exogenous nucleic acids are well known in the art. See, e.g., Sambrook et al. (2001, Molecular Cloning: A Laboratory Manual, Cold Spring Harbor Laboratory, New York). The preferred method for introducing polynucleotides into host cells is calcium phosphate transfection.

Biological methods for introducing polynucleotides of interest into host cells include the use of DNA and RNA vectors. Viral vectors, especially retroviral vectors, have become the most widely used method of inserting genes into mammalian, e.g., human cells. Other viral vectors may be derived from lentiviruses, poxviruses, herpes simplex virus I, adenoviruses and adeno-associated viruses, etc. See, e.g., US Patent Nos. 5,350,674 and 5,585,362.

Chemical means of introducing polynucleotides into host cells include colloidal dispersion systems, such as macromolecular complexes, nanocapsules, microspheres, beads; and lipid-based systems, including oil-in-water emulsions, micelles, mixed micelles, and lipid plastids. Exemplary colloidal systems for use as in-vitro and in-vivo delivery vehicles are liposomes (e.g., artificial membrane vesicles).

In the case where non-viral delivery systems are used, exemplary delivery vehicles are liposomes. The use of lipid formulations is contemplated for introducing a nucleic acid into the host cell (in vitro, ex vivo, or in vivo). In another aspect, the nucleic acid may be associated with lipids. The nucleic acid associated with lipids may be encapsulated into the aqueous interior of liposomes, dispersed in the lipid bilayer of liposomes, attached to liposomes via a linker molecule associated with both the liposomes and oligonucleotides, entrapped in liposomes, complexed with liposomes, dispersed in lipid-containing solutions, mixed with lipids, associated with lipids, contained in lipids as a suspension, contained in micelles or complexed with micelles, or associated with lipids in other ways. The lipid, lipid/DNA or lipid/expression vector associated with the composition are not limited to any particular structure in a solution. For example, they may exist in bilayer structures, as micelles or have a "collapsed" structure. They may also simply be dispersed in a solution, possibly forming aggregates that are not uniform in size or shape. Lipids are fatty substances which may be naturally occurring or synthetic lipids. For example, lipids include lipid droplets, which occur naturally in the cytoplasm as well as in such compounds including long chain aliphatic hydrocarbons and their derivatives such as fatty acids, alcohols, amines, amino alcohols and aldehydes.

In a preferred embodiment of the present application, the vector is a lentiviral vector.

### Formulation

The present application provides a formulation containing the chimeric antigen receptor CAR according to the first aspect of the present application, the nucleic acid molecule according to the second aspect of the present application, the vector according to the third aspect of the present application, or the host cell according to the fourth aspect of the present application, or the engineered immune cell according to the fifth aspect of the present application, and a pharmaceutically acceptable carrier, diluent or excipient. In one embodiment, the formulation is a liquid formulation. Preferably, the formulation is an injection. Preferably, the concentration of the CAR-T cells in the formulation is 1×10³-1×10⁸ cells/ml, and more preferably 1×10⁴-1×10⁷ cells/ml.

In one embodiment, the formulation may include a buffer such as neutral buffered saline, sulfate buffered saline, etc.; carbohydrates such as glucose, mannose, sucrose or dextran, mannitol; proteins; polypeptides or amino acids such as glycine; antioxidants; chelating agents such as EDTA or glutathione; adjuvants (e.g., aluminum hydroxide); and preservatives. The formulations of the present application are preferably formulated for intravenous administration.

### Therapeutic use

The present application includes therapeutic use of cells (e.g., T cells) transduced with lentiviral vectors (LVs) encoding the expression kit of the present application. The transduced T cells may target the tumor cell marker TSHR, synergistically activate T cells, and cause immune responses of immune cells, thereby significantly improving their killing efficiency against tumor cells.

Accordingly, the present application also provides a method of stimulating immune response mediated by target cell population of a mammal or T cells of tissues, including the following step: administering the CAR-cell of the present application to the mammal.

In one embodiment, the present application includes a cell therapy: isolating autologous T cells of a patient (or allogeneic donor), activating and performing genetical modification to produce CAR-T cells, then infusing into the body of the same patient. In this way, the probability of suffering from graft-versus-host disease is extremely low, and the antigen is recognized by T cells in a non-MHC-restricted way. In addition, one CAR-T cell may treat all cancers that express this antigen. Unlike antibody therapies, CAR-T cells may replicate in vivo, resulting in long-term persistence enabling sustained tumor control.

In one embodiment, the CAR-T cells of the present application can undergo robust in vivo expansion of T cells and persist for an extended period of time. Additionally, a CAR-mediated immune response may be a part of an adoptive immunotherapy step in which CAR-modified T cells induce an immune response specific to the antigen binding domain in the CAR. For example, the CAR-T cells targeting TSHR induce specific immune response against TSHR-expressing cells.

Although the data disclosed herein specifically discloses lentiviral vectors including TSH protein α subunit and TSH protein β subunit, hinge region and transmembrane region, and CD28 or 4-1BB and CD3ζ signaling domains which are connected in tandem, the present application should be construed as including any variation in the number of each of the components of the construct.

Cancers that can be treated include tumors that are not vascularized or not substantially vascularized, as well as tumors that are vascularized. Cancers may include non-solid tumors (such as hematological tumors, e.g., leukemias and lymphomas) or may include solid tumors. Cancer types that are treated with the CAR of the present application include but are not limited to carcinomas, blastomas, and sarcomas, and certain leukemic or lymphoid malignancies, benign and malignant tumors, and malignant masses such as sarcomas, carcinomas, and melanomas. Adult tumors/cancers and childhood tumors/cancers are also included.

The CAR-modified T cells of the present application may also be used as a type of vaccine for ex vivo immunization and/or in vivo therapy of mammals. Preferably, the mammal is a human.

For ex vivo immunization, at least one of the following occurs in vitro prior to administering the cells into a mammal: i) expanding cells, ii) introducing the nucleic acid coding the CAR into the cells, and/or iii) cryopreserving the cells.

Ex vivo procedures are well known in the art and are discussed more fully below. Briefly, cells are isolated from mammals (preferably humans) and genetically modified (i.e., transduced or transfected in vitro) with a vector expressing the CAR disclosed herein. The CAR-modified cells may be administered to mammalian recipients to provide therapeutic benefits. The mammalian recipient may be human, and the CAR-modified cells may be autologous to the recipient. Optionally, the cells may be allogeneic, syngeneic or xenogeneic to the recipient.

In addition to using cell-based vaccines for ex vivo immunization, the present application also provides compositions and methods for in vivo immunization to induce an immune response against antigens in patients.

The present application provides methods of treating tumors, including administering to a subject in need thereof a therapeutically effective amount of the CAR-modified T cells of the present application.

The CAR-modified T cells of the present application may be administered alone or as a pharmaceutical composition used in combination with diluents and/or with other components such as IL-2, IL-17 or other cytokines or cell populations. Briefly, the pharmaceutical compositions of the present application may include the target cell populations as described herein, in combination with one or more pharmaceutically or physiologically acceptable carriers, diluents or excipients. Such compositions may include buffers such as neutral buffered saline, sulfate buffered saline, and the like; carbohydrates such as glucose, mannose, sucrose or dextran, mannitol; proteins; polypeptides or amino acids such as glycine; antioxidants; chelates such as EDTA or glutathione; adjuvants (e.g., aluminum hydroxide); and preservatives. The compositions of the present application are preferably formulated for intravenous administration.

The pharmaceutical compositions of the present application may be administered in a manner appropriate to the disease to be treated (or prevented). The amount and frequency of administration will be determined by factors such as the patient's condition, and the type and severity of the patient's disease, although appropriate dosages may be determined by clinical trials.

When referring to an "effective amount", "immunologically effective amount," "effective amount against tumors," "effective tumor-suppressing amount" or "therapeutic amount," the precise amounts of the composition of the present application to be administered may be determined by physicians, taking into account the individual differences of patients (subjects) in terms of age, weight, tumor size, degree of infection or metastasis, and medical condition. It may generally be noted that the pharmaceutical composition containing the T cells described herein may be administered at the dosage of 10⁴ to 10⁹ cells/kg body weight, preferably 10⁵ to 10⁶ cells/kg body weight (including all integer values within those ranges). The T cell composition may also be administered multiple times at these doses. The cells may be administered using infusion techniques well known in immunotherapy (see, e.g., Rosenberg et al., NewEng. J. of Med. 319: 1676, 1988). The optimal dosage and treatment regimen for a particular patient may be easily determined by those skilled in the medical art by monitoring the patient for signs of disease and adjusting treatment accordingly.

Administration of the composition to subjects may be carried out in any convenient way, including nebulization, injection, swallowing, infusion, implantation, or transplantation. The compositions described herein may be administered to patients subcutaneously, intradermally, intratumorally, intranodally, intraspinally, intramuscularly, by intravenous (i.v.) injection, or intraperitoneally. In one embodiment, the T cell composition of the present application is administered to a patient by intradermal or subcutaneous injection. In another embodiment, the T cell composition of the present application is preferably administered by i.v. injection. The T cell composition may be injected directly into tumors, lymph nodes or sites of infection.

In some embodiments of the present application, the cells activated and expanded to a therapeutic level by using the methods described herein or other methods for expanding T-cells known in the art, and administrated to the patient (e.g., before, concurrently or after) in combination with any number of relevant therapeutic modalities, the therapeutic modalities including but not limited to treatment with following agents: the agents such as antiviral therapy, cidofovir and interleukin-2, cytarabine (also known as ARA-C) or natalizumab therapy for MS patients or elfazizumab therapy for psoriasis patients or other treatments for a particular tumor patient. In a further embodiment, the T cells of the present application may be used in combination with the following: chemotherapy, radiation, immunosuppressive agents such as cyclosporine, azathioprine, methotrexate, mycophenolate mofetil and FK506, antibodies or other immunotherapeutic agents. In a further embodiment, the cell composition of the present application is administered to patients (e.g., before, concurrently or after) in combination with bone marrow transplantation, use of chemotherapeutic agents such as fludarabine, external beam radiation therapy (XRT), and cyclophosphamide. For example, in one embodiment, the subject may undergo standard treatment with high dose chemotherapy followed by peripheral blood stem cell transplantation. In some embodiments, after transplantation, the subject receives infusions of the expanded immune cells of the present application. In an additional embodiment, the expanded cells are administered before surgery or after surgery.

The dosages of the above treatments administered to patients will vary depending on the precise nature of the condition being treated and the recipients of the treatments. Dosage ratios for human administration may be implemented in accordance with the accepted practice in the art. Typically, for each treatment or each course of treatment, 1×10⁶ to 1×10¹⁰ CAR-T cells of the present application may be administered to a patient, e.g., by means of intravenous reinfusion.

### Main advantages of the present application include:

1) The CAR of the present application is constructed based on a natural TSH protein, has no immunogenicity, and thus can effectively avoid the immunogenicity problem of CAR molecules and better promote the survival of CAR-T cells in the body.
2) The present application utilizes the specific tandem structure of TSH protein αβ subunits to construct a CAR that can specifically target TSHR and has good killing performance.

The present application is further described by referring to some embodiments. It should be understood that these embodiments are only used to illustrate the present application but not to limit the scope of the present application. In the following embodiments where no conditions are specified for the experimental method, conventional conditions are generally used, for example, the conditions described in Sambrook et al., Molecular Cloning: A Laboratory Manual (New York: Cold Spring Harbor Laboratory Press, 1989), or recommendations of the manufacturers. Unless otherwise specified, percentages and parts are weight percentages and weight parts.

### Materials and methods

### Plasmid construction

The cDNA coding sequences of two human TSH subunits, TSHα (NM_001303177.1, aa25-116) and TSHβ (NM_000549.5, aa21-138) were synthesized by using gene synthesis technology. The two subunits were connected by a (G4S)₃ sequence to obtain TSHαβ (connected in the TSHα-TSHβ sequence) or TSHβα (connected in the TSHβ-TSHα sequence), which were cloned into the SFG retroviral vector backbone after being double digested with Ncol and Mlul, to construct a SFG-TSHαβ-Flag-CD8-CD28-CD3ζ or SFG-TSHap-Flag-CD8-4-1BB-CD34 (TSHαβ-CAR) vector, or SFG-TSHβα-Flag-CD8-CD28-CD3ζ (TSHβα-CAR), where CD8 represented the hinge region sequence and transmembrane domain coding sequence of CD8α, CD28 was the intracellular costimulatory domain coding sequence of CD28, 4-1BB was the intracellular costimulatory domain coding sequence of 4-1BB, CD3ζ was the intracellular domain coding sequence of CD3ζ, a Flag was added between TSHαβ or TSHβα and the hinge region of CD8α to detect CAR expression (FIG. 2A and FIG. 3A).

### Construction of truncated mutant plasmid

With the TSHαβ-CAR plasmid vector used as a template, a primer was designed to remove the corresponding amino acids at the C-terminus of the TSHa subunit or TSHβ subunit, and their coding sequences were amplified, which were constructed into the TSHαβ-CAR vector backbone after being double digested with Ncol and Mlul, to replace the full-length TSHa or TSHβ subunit to construct a truncated mutant vector.

### Preparation of retrovirus expressing the TSHαβ-CAR or TSHβα-CAR

2×10⁶ 293T cells were seeded in a 10 cm dish one day in advance, cultured for 16 h, and used for virus packaging after the cells adhered. The specific steps were as follows: 30 µL of GeneJuice transfection reagent was added to 470 µL of serum-free IMDM medium, mixed evenly, and incubated at room temperature for 5 min; during this period, a 1.5 mL EP tube was prepared, and 4 µg of TSHαβ-CAR or TSHβα-CAR plasmid, 4 µg of PeqPam-3, and 2 µg of RDF packaging plasmid were mixed; after the incubation was ended, the IMDM containing the GeneJuice transfection reagent was added to the plasmid tube, mixed thoroughly, and incubated at room temperature for 15 min; after it was ended, the mixture was evenly added dropwise to the 293T cell culture dish and cultured continuously. After 48 h, the virus-containing culture medium supernatant was collected, filtered through a 0.45 µm filter membrane, and aliquoted and frozen at -80°C for later use; and 12 mL of fresh IMDM complete medium was added again to the culture dish for continuous culturing, and after 24 h, the 72 h virus supernatant was collected, filtered, aliquoted, and frozen at -80°C for later use.

### CAR-T cell preparation and in-vitro expansion

PBMC lymphocytes were isolated from the peripheral blood of healthy volunteers, the isolated lymphocytes were added at a concentration of 1×10⁶ cells per well to a 24-well plate coated with 1 µg/mL CD3 and CD28 antibodies, IL7 (10 ng/mL) and IL15 (5 ng/mL) were added after 24 h of stimulation for further 24 h of stimulation, then the cells were collected and counted, and the CAR-T cells were prepared by viral infection. The specific experimental steps were as follows: 1 mL of viral supernatant expressing the TSHαβ-CAR or TSHβα-CAR was taken and added to a 24-well plate which was coated with RetroNectin one day in advance, and centrifuged at 2000 g for 1.5 h; the supernatant was discarded after the centrifugation was ended, and 5×10⁵ activated T cells were added to each well; the culture plate was placed in a 37°C incubator for culturing for 1 h, and then centrifuged at 1000 g for 10 min, and placed in a 37°C, 5% CO₂ incubator after the centrifugation for further culturing; fresh medium was replaced after 72 h and then the medium was replaced every two days; and 5×10⁵ cells were collected four days after viral infection, and stained with anti-Flag antibody, and detected by a flow cytometry for the viral infection efficiency and CAR molecule expression (FIG. 2B-C and FIG. 3B-C), and 12 days later, CAR-T cells were collected for subsequent in-vitro experiment to detect the CAR-T cell killing function.

### Construction of TSHR-overexpressing target cell

Previous literature reports indicate that TSHR is highly expressed in thyroid cancer tissues, but protein-level expression is nearly undetectable in existing thyroid cancer cell lines. Therefore, current in-vitro studies targeting TSHR have all employed overexpression manner to construct target cell models. The present inventors examined the expression of TSHR in the thyroid cancer cell lines KTC-1 and FTC-133, but failed to detect the protein-level expression. Therefore, the lentiviral infection manner was used to overexpress an exogenous TSHR gene in the cells above. With the tumor cell cDNA used as a template, the cDNA coding sequence of the TSHR gene was amplified by PCR and cloned into the pCDH-CMV-MCS-IRES-GFP vector through double digestion with Xbal and EcoRI, and the virus was packaged in 293T cells to infect the thyroid cancer cell lines KTC-1 and FTC-133 respectively, to construct target cell strains stably overexpressing TSHR, and simultaneously a control cell strain expressing only GFP was constructed (FIG. 1).

### In-vitro coculture detection of killing function of CAR-T cell

Tumor cells were inoculated one day in advance and seeded into 24-well plate at 2.5×10⁵ cells per well for culturing overnight; after the cells adhered, 5×10⁴ CAR⁺ TSHαβ -CAR-T cells were added to each well at an effector-target ratio of 1:5, and NT (Non-transduced ) cells were added to the control group, with the cell number being the same as the total cell number in the experimental group; and after 5 days of coculturing, all T cells and tumor cells were collected, T cells were labeled with CD3, tumor cells were indicated with GFP, and dead cells were removed with ZombieAqua Dye (Biolegend), and the ratio of T cells to residual tumor cells was detected by the flow cytometry.

### ELISA detection of cytokine release

In the above coculture killing experiment, the culture medium supernatant was collected at 24 h of coculturing, and by using ELISA kit (Mabtech) for detecting IFN-γ and IL2, the release amounts of these two cytokines in the coculture supernatant were measured according to the operation steps of instructions of the kit.

### Flow cytometry

In the coculture system, T cells were labeled with APC-CD3 antibodies, and tumor cells were labeled with GFP; and the CAR molecule expression was obtained by collecting, through a BD FACSCanto II flow cytometer, data of samples labelled by APC-anti-Flag antibodies, where 10000 live cells were collected for each sample, and analyzing the data through FlowJo 10 software.

### Example 1 Construction of TSHαβ-CAR-T and TSHβα-CAR-T cells and detection of killing effects thereof

Based on the principle of specific binding of receptor and ligand, the two subunits of TSH, TSHα (CGA) and TSHβ were expressed in tandem through the (G4S)₃ sequence, and connected in tandem with the hinge region and transmembrane region of CD8α, the intracellular costimulatory domain of CD28 or the intracellular costimulatory domain of 4-1BB, the intracellular domain of CD3ζ, to construct second-generation CARs specifically targeting TSHR (TSHαβ-CAR: TSHαβ-CD28ζ-CAR and TSHβα-CAR: TSHβα-CD28ζ-CAR ) (FIG. 2A), and CAR-T cells were prepared by viral packaging and infection of lymphocytes isolated from the peripheral blood of healthy volunteers. The flow cytometry results showed that retroviruses expressing the TSHαβ-CAR and TSHβα-CAR could effectively infect T cells and express CAR molecules, successfully preparing CAR-T cells (FIG. 2B). The coculture results showed that TSHαβ-CAR-T could effectively kill tumor cells, while TSHβα-CAR-T had no obvious killing effect (Figure 2C).

### Example 2 Killing ability and specificity of TSHαβ-CAR-T cell

To further detect the ability of TSHαβ-CAR-T cells to targetedly killing TSHR-positive tumor cells, the control group and the TSHR-overexpressing cell strains KTC-1 and FTC-133 were cocultured with NT and TSHαβ-CAR-T cells at a ratio of CAR⁺ T cells to tumor cells of 1:5. After 5 days of coculturing, all T cells and tumor cells were collected, the T cells were labeled with an APC-fluorescein-conjugated CD3 antibody, and the proportion of residual tumor cells was detected by the flow cytometry. As shown in the figure, after 5 days of coculturing, TSHαβ-CAR-T cells could effectively eliminate TSHR-positive tumor cells (FIG. 4), accompanied by the release of large amounts of the cytokines IFN-γ and IL-2, but had no killing effect on the cells not expressing TSHR in the control group, and no cytokine release was detected (FIG. 5). The results demonstrate that TSHαβ-CAR-T cells can specifically recognize and kill TSHR-positive thyroid cancer cells.

### Example 3: Killing ability of truncated mutant TSHαβ-CAR-T cell

By analyzing the data of natural TSH-TSHR binding structure (complex) from NCBI, it was found that the CGA subunit and TSHβ subunit each had multiple sites to interact with TSHR, and that multiple consecutive amino acids at the C-termini of both subunits interact with each other and interact with TSHR. In this example, different truncated mutants were constructed to explore the effect of truncation on the killing function of TSHαβ-CAR-T.

The structures of the various truncated mutants are as shown in FIG. 6A. The detection results of CAR expression efficiency and killing ability are as shown in FIGs 6B and 6C, showing that the truncation mutations of TSHα and TSHβ both do not affect the functions of the CGA and TSHβ subunits, and they can form a functional complex, recognize TSHR, and kill tumor cells.

### Discussion

It is verified through experiments that the two subunits of human TSH, TSHα and TSHβ, cannot bind to the TSHR when connected in the TSHβ-TSHα sequence. However, unexpectedly, after being connected in the specific TSHβ-TSHα sequence, these two subunits can function as the extracellular antigen-binding domain of a chimeric antigen receptor to bind to the TSHR, enabling CAR-T cells to specifically recognize and kill TSHR-positive target cells. Furthermore, the truncation mutation of TSHα and TSHβ (e.g., truncation mutation of C-terminus) does not affect the recognition and binding of the antigen-binding domain to TSHR, and CAR-T cells containing the truncated mutants can still recognize and kill tumor cells.

All documents mentioned in the present application are incorporated herein as reference, just as if each document were incorporated herein as reference individually. It should also be understood that after reading the above teachings of the present application, those skilled in the art may make various changes or modifications to the present application, and that such equivalents also fall within the scope of the claims appended hereto.

### Sequences of the present application

CGA (aa25-116) coding sequence: GCTCCTGATGTGCAGGATTGCCCAGAATGCACGCTACAGGAAAACCCATTCTTCTCCCAG 
CCGGGTGCCCCAATACTTCAGTGCATGGGCTGCTGCTTCTCTAGAGCATATCCCACTCCACTAAGGT
CCAAGAAGACGATGTTGGTCCAAAAGAACGTCACCTCAGAGTCCACTTGCTGTGTAGCTAAATCATAT
AACAGGGTCACAGTAATGGGGGGTTTCAAAGTGGAGAACCACACGGCGTGCCACTGCAGTACTTGT
TATTATCACAAATCT (SEQ ID NO: 1)
CGA (aa25-116) amino acid sequence: 
APDVQDCPECTLQENPFFSQPGAPILQCMGCCFSRAYPTPLRSKKTMLVQKNVTSESTCCVA
KSYNRVTVMGGFKVENHTACHCSTCYYHKS (SEQ ID NO: 2)
TSHβ (aa21-138) coding sequence: 
TTTTGTATTCCAACTGAGTATACAATGCACATCGAAAGGAGAGAGTGTGCTTATTGCCTAAC
CATCAACACCACCATCTGTGCTGGATATTGTATGACACGGGATATCAATGGCAAACTGTTTCTTCCCA
AATATGCTCTGTCCCAGGATGTTTGCACATATAGAGACTTCATCTACAGGACTGTAGAAATACCAGGAT
GCCCACTCCATGTTGCTCCCTATTTTTCCTATCCTGTTGCTTTAAGCTGTAAGTGTGGCAAGTGCAATA
CTGACTATAGTGACTGCATACATGAAGCCATCAAGACAAACTACTGTACCAAACCTCAGAAGTCTTATC
TGGTAGGATTTTCTGTC (SEQ ID NO: 3)
TSHβ (aa21-138) amino acid sequence: 
FCIPTEYTMHIERRECAYCLTINTTICAGYCMTRDINGKLFLPKYALSQDVCTYRDFIYRTVEIPG

CPLHVAPYFSYPVALSCKCGKCNTDYSDCIHEAIKTNYCTKPQKSYLVGFSV (SEQ ID NO: 4)
CD8α hinge region and transmembrane region coding sequence: 
ACCACGACGCCAGCGCCGCGACCACCAACACCGGCGCCCACCATCGCGTCGCAGCCCC
TGTCCCTGCGCCCAGAGGCGTGCCGGCCAGCGGCGGGGGGCGCAGTGCACACGAGGGGGCTGG
ACTTCGCCTGTGATATCTACATCTGGGCGCCCTTGGCCGGGACTTGTGGGGTCCTTCTCCTGTCACT
GGTTATCACCCTTTACTGC (SEQ ID NO: 5)
CD8α hinge region and transmembrane region amino acid sequence: 
TTTPAPRPPTPAPTIASQPLSLRPEACRPAAGGAVHTRGLDFACDIYIWAPLAGTCGVLLLSLVI
TLYC (SEQ ID NO: 6)
CD28 intracellular costimulatory domain coding sequence: 
AGGAGTAAGAGGAGCAGGCTCCTGCACAGTGACTACATGAACATGACTCCCCGCCGCCC
CGGGCCCACCCGCAAGCATTACCAGCCCTATGCCCCACCACGCGACTTCGCAGCCTATCGCTCC
(SEQ ID NO: 7)
CD28 intracellular costimulatory domain amino acid sequence:
   RSKRSRLLHSDYMNMTPRRPGPTRKHYQPYAPPRDFAAYRS (SEQ ID NO: 8)
4-1BB intracellular costimulatory domain coding sequence: 
AAACGGGGCAGAAAGAAACTCCTGTATATATTCAAACAACCATTTATGAGACCAGTACAAAC
TACTCAAGAGGAAGATGGCTGTAGCTGCCGATTTCCAGAAGAAGAAGAAGGAGGATGTGAACTG
(SEQ ID NO: 9)
4-1BB intracellular costimulatory domain amino acid sequence:
   KRGRKKLLYIFKQPFMRPVQTTQEEDGCSCRFPEEEEGGCEL (SEQ ID NO: 10)
CD3ζ intracellular domain coding sequence: 
AGAGTGAAGTTCAGCAGGAGCGCAGACGCCCCCGCGTACCAGCAGGGCCAGAACCAGC
TCTATAACGAGCTCAATCTAGGACGAAGAGAGGAGTACGATGTTTTGGACAAGAGACGTGGCCGGG
ACCCTGAGATGGGGGGAAAGCCGAGAAGGAAGAACCCTCAGGAAGGCCTGTACAATGAACTGCAG
AAAGATAAGATGGCGGAGGCCTACAGTGAGATTGGGATGAAAGGCGAGCGCCGGAGGGGCAAGGG
GCACGATGGCCTTTACCAGGGTCTCAGTACAGCCACCAAGGACACCTACGACGCCCTTCACATGCA
GGCCCTGCCCCCTCGCTAA (SEQ ID NO: 11)
CD3ζ intracellular domain amino acid sequence: 
RVKFSRSADAPAYQQGQNQLYNELNLGRREEYDVLDKRRGRDPEMGGKPRRKNPQEGLYN
ELQKDKMAEAYSEIGMKGERRRGKGHDGLYQGLSTATKDTYDALHMQALPPR (SEQ ID NO: 12)
TSHαβ-CD8α-CD28-CD3ζ coding sequence: 
ATGGAATTCGGCCTGAGCTGGCTGTTCCTGGTGGCCATCCTGAAGGGCGTGCAGTGCGC
TCCTGATGTGCAGGATTGCCCAGAATGCACGCTACAGGAAAACCCATTCTTCTCCCAGCCGGGTGC
CCCAATACTTCAGTGCATGGGCTGCTGCTTCTCTAGAGCATATCCCACTCCACTAAGGTCCAAGAAG
ACGATGTTGGTCCAAAAGAACGTCACCTCAGAGTCCACTTGCTGTGTAGCTAAATCATATAACAGGGT
CACAGTAATGGGGGGTTTCAAAGTGGAGAACCACACGGCGTGCCACTGCAGTACTTGTTATTATCAC
AAATCTGGCGGCGGAGGATCTGGCGGAGGCGGAAGTGGCGGAGGGGGCTCTTTTTGTATTCCAAC
TGAGTATACAATGCACATCGAAAGGAGAGAGTGTGCTTATTGCCTAACCATCAACACCACCATCTGTG
CTGGATATTGTATGACACGGGATATCAATGGCAAACTGTTTCTTCCCAAATATGCTCTGTCCCAGGATG
TTTGCACATATAGAGACTTCATCTACAGGACTGTAGAAATACCAGGATGCCCACTCCATGTTGCTCCC
TATTTTTCCTATCCTGTTGCTTTAAGCTGTAAGTGTGGCAAGTGCAATACTGACTATAGTGACTGCATA
CATGAAGCCATCAAGACAAACTACTGTACCAAACCTCAGAAGTCTTATCTGGTAGGATTTTCTGTCAC
GCGTGGATCCGACTACAAGGATGACGACGACAAGGGTGGTACCACGACGCCAGCGCCGCGACCAC
 
CAACACCGGCGCCCACCATCGCGTCGCAGCCCCTGTCCCTGCGCCCAGAGGCGTGCCGGCCAGC
GGCGGGGGGCGCAGTGCACACGAGGGGGCTGGACTTCGCCTGTGATATCTACATCTGGGCGCCCT
TGGCCGGGACTTGTGGGGTCCTTCTCCTGTCACTGGTTATCACCCTTTACTGCAGGAGTAAGAGGA
GCAGGCTCCTGCACAGTGACTACATGAACATGACTCCCCGCCGCCCCGGGCCCACCCGCAAGCATT
ACCAGCCCTATGCCCCACCACGCGACTTCGCAGCCTATCGCTCCAGAGTGAAGTTCAGCAGGAGCG
CAGACGCCCCCGCGTACCAGCAGGGCCAGAACCAGCTCTATAACGAGCTCAATCTAGGACGAAGAG
AGGAGTACGATGTTTTGGACAAGAGACGTGGCCGGGACCCTGAGATGGGGGGAAAGCCGAGAAGG
AAGAACCCTCAGGAAGGCCTGTACAATGAACTGCAGAAAGATAAGATGGCGGAGGCCTACAGTGAG
ATTGGGATGAAAGGCGAGCGCCGGAGGGGCAAGGGGCACGATGGCCTTTACCAGGGTCTCAGTAC
AGCCACCAAGGACACCTACGACGCCCTTCACATGCAGGCCCTGCCCCCTCGCTAA (SEQ ID NO: 13)
TSHαβ-CD8α-CD28-CD3ζ amino acid sequence: 
MEFGLSWLFLVAILKGVQCAPDVQDCPECTLQENPFFSQPGAPILQCMGCCFSRAYPTPLRS
KKTMLVQKNVTSESTCCVAKSYNRVTVMGGFKVENHTACHCSTCYYHKSGGGGSGGGGSGGGGSFCI
PTEYTMHIERRECAYCLTINTTICAGYCMTRDINGKLFLPKYALSQDVCTYRDFIYRTVEIPGCPLHVAPYF
SYPVALSCKCGKCNTDYSDCIHEAIKTNYCTKPQKSYLVGFSVTRGSDYKDDDDKGGTTTPAPRPPTPA
PTIASQPLSLRPEACRPAAGGAVHTRGLDFACDIYIWAPLAGTCGVLLLSLVITLYCRSKRSRLLHSDYMN
MTPRRPGPTRKHYQPYAPPRDFAAYRSRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTATKDTYDALHMQ
ALPPR (SEQ ID NO: 14)
TSHαβ-CD8α-4-1BB-CD3ζ coding sequence: 
ATGGAATTCGGCCTGAGCTGGCTGTTCCTGGTGGCCATCCTGAAGGGCGTGCAGTGCGC
TCCTGATGTGCAGGATTGCCCAGAATGCACGCTACAGGAAAACCCATTCTTCTCCCAGCCGGGTGC
CCCAATACTTCAGTGCATGGGCTGCTGCTTCTCTAGAGCATATCCCACTCCACTAAGGTCCAAGAAG
ACGATGTTGGTCCAAAAGAACGTCACCTCAGAGTCCACTTGCTGTGTAGCTAAATCATATAACAGGGT
CACAGTAATGGGGGGTTTCAAAGTGGAGAACCACACGGCGTGCCACTGCAGTACTTGTTATTATCAC
AAATCTGGCGGCGGAGGATCTGGCGGAGGCGGAAGTGGCGGAGGGGGCTCTTTTTGTATTCCAAC
TGAGTATACAATGCACATCGAAAGGAGAGAGTGTGCTTATTGCCTAACCATCAACACCACCATCTGTG
CTGGATATTGTATGACACGGGATATCAATGGCAAACTGTTTCTTCCCAAATATGCTCTGTCCCAGGATG
TTTGCACATATAGAGACTTCATCTACAGGACTGTAGAAATACCAGGATGCCCACTCCATGTTGCTCCC
TATTTTTCCTATCCTGTTGCTTTAAGCTGTAAGTGTGGCAAGTGCAATACTGACTATAGTGACTGCATA
 
CATGAAGCCATCAAGACAAACTACTGTACCAAACCTCAGAAGTCTTATCTGGTAGGATTTTCTGTCAC
GCGTGGATCCGACTACAAGGATGACGACGACAAGGGTGGTACCACGACGCCAGCGCCGCGACCAC
CAACACCGGCGCCCACCATCGCGTCGCAGCCCCTGTCCCTGCGCCCAGAGGCGTGCCGGCCAGC
GGCGGGGGGCGCAGTGCACACGAGGGGGCTGGACTTCGCCTGTGATATCTACATCTGGGCGCCCT
TGGCCGGGACTTGTGGGGTCCTTCTCCTGTCACTGGTTATCACCCTTTACTGCAAACGGGGCAGAA
AGAAACTCCTGTATATATTCAAACAACCATTTATGAGACCAGTACAAACTACTCAAGAGGAAGATGGCT
GTAGCTGCCGATTTCCAGAAGAAGAAGAAGGAGGATGTGAACTGAGAGTGAAGTTCAGCAGGAGCG
CAGACGCCCCCGCGTACCAGCAGGGCCAGAACCAGCTCTATAACGAGCTCAATCTAGGACGAAGAG
AGGAGTACGATGTTTTGGACAAGAGACGTGGCCGGGACCCTGAGATGGGGGGAAAGCCGAGAAGG
AAGAACCCTCAGGAAGGCCTGTACAATGAACTGCAGAAAGATAAGATGGCGGAGGCCTACAGTGAG
ATTGGGATGAAAGGCGAGCGCCGGAGGGGCAAGGGGCACGATGGCCTTTACCAGGGTCTCAGTAC
AGCCACCAAGGACACCTACGACGCCCTTCACATGCAGGCCCTGCCCCCTCGCTAA (SEQ ID NO: 15)
TSHαβ-CD8α-4-1BB-CD3ζ amino acid sequence: 
MEFGLSWLFLVAILKGVQCAPDVQDCPECTLQENPFFSQPGAPILQCMGCCFSRAYPTPLRS
KKTMLVQKNVTSESTCCVAKSYNRVTVMGGFKVENHTACHCSTCYYHKSGGGGSGGGGSGGGGSFCI
PTEYTMHIERRECAYCLTINTTICAGYCMTRDINGKLFLPKYALSQDVCTYRDFIYRTVEIPGCPLHVAPYF
SYPVALSCKCGKCNTDYSDCIHEAIKTNYCTKPQKSYLVGFSVTRGSDYKDDDDKGGTTTPAPRPPTPA
PTIASQPLSLRPEACRPAAGGAVHTRGLDFACDIYIWAPLAGTCGVLLLSLVITLYCKRGRKKLLYIFKQPF
MRPVQTTQEEDGCSCRFPEEEEGGCELRVKFSRSADAPAYQQGQNQLYNELNLGRREEYDVLDKRRG
RDPEMGGKPRRKNPQEGLYNELQKDKMAEAYSEIGMKGERRRGKGHDGLYQGLSTATKDTYDALHMQ
ALPPR (SEQ ID NO: 16)
TSHβα-CD8α-CD28-CD3ζ coding sequence: 
ATGGAATTCGGCCTGAGCTGGCTGTTCCTGGTGGCCATCCTGAAGGGCGTGCAGTGCTT
TTGTATTCCAACTGAGTATACAATGCACATCGAAAGGAGAGAGTGTGCTTATTGCCTAACCATCAACA
CCACCATCTGTGCTGGATATTGTATGACACGGGATATCAATGGCAAACTGTTTCTTCCCAAATATGCTC
TGTCCCAGGATGTTTGCACATATAGAGACTTCATCTACAGGACTGTAGAAATACCAGGATGCCCACTC
CATGTTGCTCCCTATTTTTCCTATCCTGTTGCTTTAAGCTGTAAGTGTGGCAAGTGCAATACTGACTAT
AGTGACTGCATACATGAAGCCATCAAGACAAACTACTGTACCAAACCTCAGAAGTCTTATCTGGTAGG
ATTTTCTGTCGGCGGCGGAGGATCTGGCGGAGGCGGAAGTGGCGGAGGGGGCTCTGCTCCTGATG
TGCAGGATTGCCCAGAATGCACGCTACAGGAAAACCCATTCTTCTCCCAGCCGGGTGCCCCAATACT
 
TCAGTGCATGGGCTGCTGCTTCTCTAGAGCATATCCCACTCCACTAAGGTCCAAGAAGACGATGTTG
GTCCAAAAGAACGTCACCTCAGAGTCCACTTGCTGTGTAGCTAAATCATATAACAGGGTCACAGTAAT
GGGGGTTTCAAAGTGGAGAACCACACGGCGTGCCACTGCAGTACTTGTTATTATCACAAATCTACG
CGTGGATCCGACTACAAGGATGACGACGACAAGGGTGGTACCACGACGCCAGCGCCGCGACCACC
AACACCGGCGCCCACCATCGCGTCGCAGCCCCTGTCCCTGCGCCCAGAGGCGTGCCGGCCAGCG
GCGGGGGGCGCAGTGCACACGAGGGGGCTGGACTTCGCCTGTGATATCTACATCTGGGCGCCCTT
GGCCGGGACTTGTGGGGTCCTTCTCCTGTCACTGGTTATCACCCTTTACTGCAGGAGTAAGAGGAG
CAGGCTCCTGCACAGTGACTACATGAACATGACTCCCCGCCGCCCCGGGCCCACCCGCAAGCATTA
CCAGCCCTATGCCCCACCACGCGACTTCGCAGCCTATCGCTCCAGAGTGAAGTTCAGCAGGAGCGC
AGACGCCCCCGCGTACCAGCAGGGCCAGAACCAGCTCTATAACGAGCTCAATCTAGGACGAAGAGA
GGAGTACGATGTTTTGGACAAGAGACGTGGCCGGGACCCTGAGATGGGGGGAAAGCCGAGAAGGA
AGAACCCTCAGGAAGGCCTGTACAATGAACTGCAGAAAGATAAGATGGCGGAGGCCTACAGTGAGA
TTGGGATGAAAGGCGAGCGCCGGAGGGGCAAGGGGCACGATGGCCTTTACCAGGGTCTCAGTACA
GCCACCAAGGACACCTACGACGCCCTTCACATGCAGGCCCTGCCCCCTCGCTAA (SEQ ID NO: 17)
TSHβα-CD8α-CD28-CD3ζ amino acid sequence: 
MEFGLSWLFLVAILKGVQCFCIPTEYTMHIERRECAYCLTINTTICAGYCMTRDINGKLFLPKYA
LSQDVCTYRDFIYRTVEIPGCPLHVAPYFSYPVALSCKCGKCNTDYSDCIHEAIKTNYCTKPQKSYLVGFS
VGGGGSGGGGSGGGGSAPDVQDCPECTLQENPFFSQPGAPILQCMGCCFSRAYPTPLRSKKTMLVQK
NVTSESTCCVAKSYNRVTVMGGFKVENHTACHCSTCYYHKSTRGSDYKDDDDKGGTTTPAPRPPTPAP
TIASQPLSLRPEACRPAAGGAVHTRGLDFACDIYIWAPLAGTCGVLLLSLVITLYCRSKRSRLLHSDYMNM
TPRRPGPTRKHYQPYAPPRDFAAYRSRVKFSRSADAPAYQQGQNQLYNELNLGRREEYDVLDKRRGRD
PEMGGKPRRKNPQEGLYNELQKDKMAEAYSEIGMKGERRRGKGHDGLYQGLSTATKDTYDALHMQAL
PPR (SEQ ID NO: 18)
Truncated TSHα (25-114) amino acid sequence: 
APDVQDCPECTLQENPFFSQPGAPILQCMGCCFSRAYPTPLRSKKTMLVQKNVTSESTCCVA

KSYNRVTVMGGFKVENHTACHCSTCYYH (SEQ ID NO: 19)
Truncated TSHβ (21-126) amino acid sequence: 
FCIPTEYTMHIERRECAYCLTINTTICAGYCMTRDINGKLFLPKYALSQDVCTYRDFIYRTVEIPG

CPLHVAPYFSYPVALSCKCGKCNTDYSDCIHEAIKTNYCT (SEQ ID NO: 20)
Truncated TSHβ (21-124) amino acid sequence: 
FCIPTEYTMHIERRECAYCLTINTTICAGYCMTRDINGKLFLPKYALSQDVCTYRDFIYRTVEIPG

CPLHVAPYFSYPVALSCKCGKCNTDYSDCIHEAIKTNY (SEQ ID NO: 21)
Truncated TSHβ (21-122) amino acid sequence: 
FCIPTEYTMHIERRECAYCLTINTTICAGYCMTRDINGKLFLPKYALSQDVCTYRDFIYRTVEIPG
CPLHVAPYFSYPVALSCKCGKCNTDYSDCIHEAIKT (SEQ ID NO: 22)
TSHαβ (21-126) CAR coding sequence: 
ATGGAATTCGGCCTGAGCTGGCTGTTCCTGGTGGCCATCCTGAAGGGCGTGCAGTGCGC
TCCTGATGTGCAGGATTGCCCAGAATGCACGCTACAGGAAAACCCATTCTTCTCCCAGCCGGGTGC
CCCAATACTTCAGTGCATGGGCTGCTGCTTCTCTAGAGCATATCCCACTCCACTAAGGTCCAAGAAG
ACGATGTTGGTCCAAAAGAACGTCACCTCAGAGTCCACTTGCTGTGTAGCTAAATCATATAACAGGGT
CACAGTAATGGGGGGTTTCAAAGTGGAGAACCACACGGCGTGCCACTGCAGTACTTGTTATTATCAC
AAATCTGGCGGCGGAGGATCTGGCGGAGGCGGAAGTGGCGGAGGGGGCTCTTTTTGTATTCCAAC
TGAGTATACAATGCACATCGAAAGGAGAGAGTGTGCTTATTGCCTAACCATCAACACCACCATCTGTG
CTGGATATTGTATGACACGGGATATCAATGGCAAACTGTTTCTTCCCAAATATGCTCTGTCCCAGGATG
TTTGCACATATAGAGACTTCATCTACAGGACTGTAGAAATACCAGGATGCCCACTCCATGTTGCTCCC
TATTTTTCCTATCCTGTTGCTTTAAGCTGTAAGTGTGGCAAGTGCAATACTGACTATAGTGACTGCATA
CATGAAGCCATCAAGACAAACTACTGTACCACGCGTGGATCCGACTACAAGGATGACGACGACAAGG
GTGGTACCACGACGCCAGCGCCGCGACCACCAACACCGGCGCCCACCATCGCGTCGCAGCCCCT
GTCCCTGCGCCCAGAGGCGTGCCGGCCAGCGGCGGGGGGCGCAGTGCACACGAGGGGGCTGGA
CTTCGCCTGTGATATCTACATCTGGGCGCCCTTGGCCGGGACTTGTGGGGTCCTTCTCCTGTCACTG
GTTATCACCCTTTACTGCAGGAGTAAGAGGAGCAGGCTCCTGCACAGTGACTACATGAACATGACTC
CCCGCCGCCCCGGGCCCACCCGCAAGCATTACCAGCCCTATGCCCCACCACGCGACTTCGCAGCC
TATCGCTCCAGAGTGAAGTTCAGCAGGAGCGCAGACGCCCCCGCGTACCAGCAGGGCCAGAACCA
GCTCTATAACGAGCTCAATCTAGGACGAAGAGAGGAGTACGATGTTTTGGACAAGAGACGTGGCCG
GGACCCTGAGATGGGGGGAAAGCCGAGAAGGAAGAACCCTCAGGAAGGCCTGTACAATGAACTGC
AGAAAGATAAGATGGCGGAGGCCTACAGTGAGATTGGGATGAAAGGCGAGCGCCGGAGGGGCAAG
GGGCACGATGGCCTTTACCAGGGTCTCAGTACAGCCACCAAGGACACCTACGACGCCCTTCACATG
CAGGCCCTGCCCCCTCGCTAA (SEQ ID NO: 23)
TSHαβ (21-126) CAR amino acid sequence: MEFGLSWLFLVAILKGVQCAPDVQDCPECTLQENPFFSQPGAPILQCMGCCFSRAYPTPLRS 
KKTMLVQKNVTSESTCCVAKSYNRVTVMGGFKVENHTACHCSTCYYHKSGGGGSGGGGSGGGGSFCI
PTEYTMHIERRECAYCLTINTTICAGYCMTRDINGKLFLPKYALSQDVCTYRDFIYRTVEIPGCPLHVAPYF
SYPVALSCKCGKCNTDYSDCIHEAIKTNYCTTRGSDYKDDDDKGGTTTPAPRPPTPAPTIASQPLSLRPE
ACRPAAGGAVHTRGLDFACDIYIWAPLAGTCGVLLLSLVITLYCRSKRSRLLHSDYMNMTPRRPGPTRKH
YQPYAPPRDFAAYRSRVKFSRSADAPAYQQGQNQLYNELNLGRREEYDVLDKRRGRDPEMGGKPRRK
NPQEGLYNELQKDKMAEAYSEIGMKGERRRGKGHDGLYQGLSTATKDTYDALHMQALPPR (SEQ ID NO:
24)
TSHαβ (21-124) CAR coding sequence: 
ATGGAATTCGGCCTGAGCTGGCTGTTCCTGGTGGCCATCCTGAAGGGCGTGCAGTGCGC
TCCTGATGTGCAGGATTGCCCAGAATGCACGCTACAGGAAAACCCATTCTTCTCCCAGCCGGGTGC
CCCAATACTTCAGTGCATGGGCTGCTGCTTCTCTAGAGCATATCCCACTCCACTAAGGTCCAAGAAG
ACGATGTTGGTCCAAAAGAACGTCACCTCAGAGTCCACTTGCTGTGTAGCTAAATCATATAACAGGGT
CACAGTAATGGGGGGTTTCAAAGTGGAGAACCACACGGCGTGCCACTGCAGTACTTGTTATTATCAC
AAATCTGGCGGCGGAGGATCTGGCGGAGGCGGAAGTGGCGGAGGGGGCTCTTTTTGTATTCCAAC
TGAGTATACAATGCACATCGAAAGGAGAGAGTGTGCTTATTGCCTAACCATCAACACCACCATCTGTG
CTGGATATTGTATGACACGGGATATCAATGGCAAACTGTTTCTTCCCAAATATGCTCTGTCCCAGGATG
TTTGCACATATAGAGACTTCATCTACAGGACTGTAGAAATACCAGGATGCCCACTCCATGTTGCTCCC
TATTTTTCCTATCCTGTTGCTTTAAGCTGTAAGTGTGGCAAGTGCAATACTGACTATAGTGACTGCATA
CATGAAGCCATCAAGACAAACTACACGCGTGGATCCGACTACAAGGATGACGACGACAAGGGTGGT
ACCACGACGCCAGCGCCGCGACCACCAACACCGGCGCCCACCATCGCGTCGCAGCCCCTGTCCCT
GCGCCCAGAGGCGTGCCGGCCAGCGGCGGGGGGCGCAGTGCACACGAGGGGGCTGGACTTCGC
CTGTGATATCTACATCTGGGCGCCCTTGGCCGGGACTTGTGGGGTCCTTCTCCTGTCACTGGTTATC
ACCCTTTACTGCAGGAGTAAGAGGAGCAGGCTCCTGCACAGTGACTACATGAACATGACTCCCCGC
CGCCCCGGGCCCACCCGCAAGCATTACCAGCCCTATGCCCCACCACGCGACTTCGCAGCCTATCGC
TCCAGAGTGAAGTTCAGCAGGAGCGCAGACGCCCCCGCGTACCAGCAGGGCCAGAACCAGCTCTA
TAACGAGCTCAATCTAGGACGAAGAGAGGAGTACGATGTTTTGGACAAGAGACGTGGCCGGGACCC
TGAGATGGGGGGAAAGCCGAGAAGGAAGAACCCTCAGGAAGGCCTGTACAATGAACTGCAGAAAG
ATAAGATGGCGGAGGCCTACAGTGAGATTGGGATGAAAGGCGAGCGCCGGAGGGGCAAGGGGCAC
GATGGCCTTTACCAGGGTCTCAGTACAGCCACCAAGGACACCTACGACGCCCTTCACATGCAGGCC
CTGCCCCCTCGCTAA (SEQ ID NO: 25)
TSHαβ (21-124) CAR amino acid sequence: 
MEFGLSWLFLVAILKGVQCAPDVQDCPECTLQENPFFSQPGAPILQCMGCCFSRAYPTPLRS
KKTMLVQKNVTSESTCCVAKSYNRVTVMGGFKVENHTACHCSTCYYHKSGGGGSGGGGSGGGGSFCI
PTEYTMHIERRECAYCLTINTTICAGYCMTRDINGKLFLPKYALSQDVCTYRDFIYRTVEIPGCPLHVAPYF
SYPVALSCKCGKCNTDYSDCIHEAIKTNYTRGSDYKDDDDKGGTTTPAPRPPTPAPTIASQPLSLRPEAC
RPAAGGAVHTRGLDFACDIYIWAPLAGTCGVLLLSLVITLYCRSKRSRLLHSDYMNMTPRRPGPTRKHYQ
PYAPPRDFAAYRSRVKFSRSADAPAYQQGQNQLYNELNLGRREEYDVLDKRRGRDPEMGGKPRRKNP
QEGLYNELQKDKMAEAYSEIGMKGERRRGKGHDGLYQGLSTATKDTYDALHMQALPPR (SEQ ID NO:
26)
**[00261]** TSHαβ (21-122) CAR coding sequence: 
ATGGAATTCGGCCTGAGCTGGCTGTTCCTGGTGGCCATCCTGAAGGGCGTGCAGTGCGC
TCCTGATGTGCAGGATTGCCCAGAATGCACGCTACAGGAAAACCCATTCTTCTCCCAGCCGGGTGC
CCCAATACTTCAGTGCATGGGCTGCTGCTTCTCTAGAGCATATCCCACTCCACTAAGGTCCAAGAAG
ACGATGTTGGTCCAAAAGAACGTCACCTCAGAGTCCACTTGCTGTGTAGCTAAATCATATAACAGGGT
CACAGTAATGGGGGGTTTCAAAGTGGAGAACCACACGGCGTGCCACTGCAGTACTTGTTATTATCAC
AAATCTGGCGGCGGAGGATCTGGCGGAGGCGGAAGTGGCGGAGGGGGCTCTTTTTGTATTCCAAC
TGAGTATACAATGCACATCGAAAGGAGAGAGTGTGCTTATTGCCTAACCATCAACACCACCATCTGTG
CTGGATATTGTATGACACGGGATATCAATGGCAAACTGTTTCTTCCCAAATATGCTCTGTCCCAGGATG
TTTGCACATATAGAGACTTCATCTACAGGACTGTAGAAATACCAGGATGCCCACTCCATGTTGCTCCC
TATTTTTCCTATCCTGTTGCTTTAAGCTGTAAGTGTGGCAAGTGCAATACTGACTATAGTGACTGCATA
CATGAAGCCATCAAGACAACGCGTGGATCCGACTACAAGGATGACGACGACAAGGGTGGTACCACG
ACGCCAGCGCCGCGACCACCAACACCGGCGCCCACCATCGCGTCGCAGCCCCTGTCCCTGCGCC
CAGAGGCGTGCCGGCCAGCGGCGGGGGGCGCAGTGCACACGAGGGGGCTGGACTTCGCCTGTG
ATATCTACATCTGGGCGCCCTTGGCCGGGACTTGTGGGGTCCTTCTCCTGTCACTGGTTATCACCCT
TTACTGCAGGAGTAAGAGGAGCAGGCTCCTGCACAGTGACTACATGAACATGACTCCCCGCCGCCC
CGGGCCCACCCGCAAGCATTACCAGCCCTATGCCCCACCACGCGACTTCGCAGCCTATCGCTCCAG
AGTGAAGTTCAGCAGGAGCGCAGACGCCCCCGCGTACCAGCAGGGCCAGAACCAGCTCTATAACG
AGCTCAATCTAGGACGAAGAGAGGAGTACGATGTTTTGGACAAGAGACGTGGCCGGGACCCTGAGA
TGGGGGGAAAGCCGAGAAGGAAGAACCCTCAGGAAGGCCTGTACAATGAACTGCAGAAAGATAAGA
TGGCGGAGGCCTACAGTGAGATTGGGATGAAAGGCGAGCGCCGGAGGGGCAAGGGGCACGATGG
 
CCTTTACCAGGGTCTCAGTACAGCCACCAAGGACACCTACGACGCCCTTCACATGCAGGCCCTGCC
CCCTCGCTAA (SEQ ID NO: 27)
TSHαβ (21-122) CAR amino acid sequence: 
MEFGLSWLFLVAILKGVQCAPDVQDCPECTLQENPFFSQPGAPILQCMGCCFSRAYPTPLRS
KKTMLVQKNVTSESTCCVAKSYNRVTVMGGFKVENHTACHCSTCYYHKSGGGGSGGGGSGGGGSFCI
PTEYTMHIERRECAYCLTINTTICAGYCMTRDINGKLFLPKYALSQDVCTYRDFIYRTVEIPGCPLHVAPYF
SYPVALSCKCGKCNTDYSDCIHEAIKTTRGSDYKDDDDKGGTTTPAPRPPTPAPTIASQPLSLRPEACRP
AAGGAVHTRGLDFACDIYIWAPLAGTCGVLLLSLVITLYCRSKRSRLLHSDYMNMTPRRPGPTRKHYQPY
APPRDFAAYRSRVKFSRSADAPAYQQGQNQLYNELNLGRREEYDVLDKRRGRDPEMGGKPRRKNPQE
GLYNELQKDKMAEAYSEIGMKGERRRGKGHDGLYQGLSTATKDTYDALHMQALPPR (SEQ ID NO: 28)
TSHα (25-114) β CAR coding sequence:

ATGGAATTCGGCCTGAGCTGGCTGTTCCTGGTGGCCATCCTGAAGGGCGTGCAGTGCGC
TCCTGATGTGCAGGATTGCCCAGAATGCACGCTACAGGAAAACCCATTCTTCTCCCAGCCGGGTGC
CCCAATACTTCAGTGCATGGGCTGCTGCTTCTCTAGAGCATATCCCACTCCACTAAGGTCCAAGAAG
ACGATGTTGGTCCAAAAGAACGTCACCTCAGAGTCCACTTGCTGTGTAGCTAAATCATATAACAGGGT
CACAGTAATGGGGGGTTTCAAAGTGGAGAACCACACGGCGTGCCACTGCAGTACTTGTTATTATCAC
GGCGGCGGAGGATCTGGCGGAGGCGGAAGTGGCGGAGGGGGCTCTTTTTGTATTCCAACTGAGTA
TACAATGCACATCGAAAGGAGAGAGTGTGCTTATTGCCTAACCATCAACACCACCATCTGTGCTGGAT
ATTGTATGACACGGGATATCAATGGCAAACTGTTTCTTCCCAAATATGCTCTGTCCCAGGATGTTTGCA
CATATAGAGACTTCATCTACAGGACTGTAGAAATACCAGGATGCCCACTCCATGTTGCTCCCTATTTTT
CCTATCCTGTTGCTTTAAGCTGTAAGTGTGGCAAGTGCAATACTGACTATAGTGACTGCATACATGAA
GCCATCAAGACAAACTACTGTACCAAACCTCAGAAGTCTTATACGCGTGGATCCGACTACAAGGATGA
CGACGACAAGGGTGGTACCACGACGCCAGCGCCGCGACCACCAACACCGGCGCCCACCATCGCG
TCGCAGCCCCTGTCCCTGCGCCCAGAGGCGTGCCGGCCAGCGGCGGGGGGCGCAGTGCACACG
AGGGGGCTGGACTTCGCCTGTGATATCTACATCTGGGCGCCCTTGGCCGGGACTTGTGGGGTCCTT
CTCCTGTCACTGGTTATCACCCTTTACTGCAGGAGTAAGAGGAGCAGGCTCCTGCACAGTGACTACA
TGAACATGACTCCCCGCCGCCCCGGGCCCACCCGCAAGCATTACCAGCCCTATGCCCCACCACGC
GACTTCGCAGCCTATCGCTCCAGAGTGAAGTTCAGCAGGAGCGCAGACGCCCCCGCGTACCAGCA
GGGCCAGAACCAGCTCTATAACGAGCTCAATCTAGGACGAAGAGAGGAGTACGATGTTTTGGACAAG
AGACGTGGCCGGGACCCTGAGATGGGGGGAAAGCCGAGAAGGAAGAACCCTCAGGAAGGCCTGT

ACAATGAACTGCAGAAAGATAAGATGGCGGAGGCCTACAGTGAGATTGGGATGAAAGGCGAGCGCC

GGAGGGGCAAGGGGCACGATGGCCTTTACCAGGGTCTCAGTACAGCCACCAAGGACACCTACGAC

GCCCTTCACATGCAGGCCCTGCCCCCTCGCTAA (SEQ ID NO: 29)

MEFGLSWLFLVAILKGVQCAPDVQDCPECTLQENPFFSQPGAPILQCMGCCFSRAYPTPLRS
KKTMLVQKNVTSESTCCVAKSYNRVTVMGGFKVENHTACHCSTCYYHGGGGSGGGGSGGGGSFCIPT
EYTMHIERRECAYCLTINTTICAGYCMTRDINGKLFLPKYALSQDVCTYRDFIYRTVEIPGCPLHVAPYFSY
PVALSCKCGKCNTDYSDCIHEAIKTNYCTKPQKSYTRGSDYKDDDDKGGTTTPAPRPPTPAPTIASQPLS
LRPEACRPAAGGAVHTRGLDFACDIYIWAPLAGTCGVLLLSLVITLYCRSKRSRLLHSDYMNMTPRRPGP
TRKHYQPYAPPRDFAAYRSRVKFSRSADAPAYQQGQNQLYNELNLGRREEYDVLDKRRGRDPEMGGK
PRRKNPQEGLYNELQKDKMAEAYSEIGMKGERRRGKGHDGLYQGLSTATKDTYDALHMQALPPR (SEQ
ID NO: 30)

Labeled protein
   TRGSDYKDDDDKGG (SEQ ID NO: 31)
Linker peptide
   GGGGSGGGGSGGGGS (SEQ ID NO: 32)

## Claims

1. A chimeric antigen receptor (CAR), wherein the CAR contains an extracellular antigen-binding domain that targets a thyroid-stimulating hormone receptor (TSHR), and the extracellular antigen-binding domain comprises a structure as shown by a following formula (I):
Tα-L-Tβ (I) ,
wherein
each "-" is independently a linker peptide or a peptide bond;
Tα is a TSH protein α subunit, or a fragment thereof;
Tβ is a TSH protein β subunit, or a fragment thereof; and
L is nothing or a linker peptide.

2. The CAR according to claim 1, wherein the fragment of the TSH protein α subunit is a fragment obtained by truncating 1-10 amino acids, preferably 1-6 amino acids, and more preferably 1-2 amino acids, from a C-terminus of the TSH protein α subunit as shown in SEQ ID NO: 2.

3. The CAR according to claim 1 or 2, wherein an amino acid sequence of the Tα is as shown in SEQ ID NO: 2 or SEQ ID NO: 19, or has a sequence identity of ≥85% (preferably ≥90%, more preferably ≥95%, for example ≥96%, 297%, ≥98% or 299%) to and has same or substantially same binding function as the sequence as shown in SEQ ID NO: 2 or SEQ ID NO: 19.

4. The CAR according to claim 1, wherein the fragment of the TSH protein β subunit is a fragment obtained by truncating 1-20 amino acids, and preferably 1-16, 1-14 or 1-12 amino acids from a C-terminus of the TSH protein β subunit as shown in SEQ ID NO: 4.

5. The CAR according to claim 1 or 4, wherein an amino acid sequence of the Tβ is as shown in SEQ ID NO: 4, 20, 21 or 22, or has a sequence identity of ≥85% (preferably 290%, more preferably 295%, for example ≥96%, ≥97%, ≥98% or 299%) to and has same or substantially same binding function as the sequence as shown in SEQ ID NO: 4, 20, 21 or 22.

6. The chimeric antigen receptor according to any one of claims 1 to 5, wherein a sequence of the L is (G4S)n, wherein n is an integer selected from 1 to 6.

7. The chimeric antigen receptor according to any one of claims 1 to 6, wherein a structure of the CAR is as shown in a following formula II:
S-EB-F-H-TM-C-CD3ζ (II)
in the formula,
each "-" is independently a linker peptide or a peptide bond;
S is nothing or a signal peptide sequence;
EB is the extracellular antigen-binding domain;
H is nothing or a hinge region;
TM is the transmembrane domain;
C is nothing or an intracellular costimulatory domain;
CD3ζ is an intracellular domain coding sequence derived from CD3ζ; and
F is nothing or a labeled protein.

8. The chimeric antigen receptor according to any one of claims 1 to 7, wherein an amino acid sequence of the chimeric antigen receptor CAR is as shown in SEQ ID NO: 14, 16, 24, 26, 28 or 30, or has a sequence identity of ≥85% (preferably 290%, more preferably ≥95%, for example ≥96%, ≥97%, ≥98% or ≥99%) to the sequence as shown in SEQ ID NO: 14, 16, 24, 26, 28 or 30.

9. A nucleic acid molecule, wherein the nucleic acid molecule encodes the chimeric antigen receptor according to any one of claims 1 to 8.

10. A vector, wherein the vector contains the nucleic acid molecule according to claim 9.

11. A host cell, wherein the host cell contains the vector according to claim 10, or is integrated, in chromosome, with the nucleic acid molecule according to claim 9 which is exogenous, or expresses the CAR according to any one of claims 1 to 8.

12. An engineered immune cell, wherein the immune cell contains the vector according to claim 10, or is integrated, in chromosome, with the nucleic acid molecule according to claim 9 which is exogenous, or expresses the CAR according to any one of claims 1 to 8.

13. The engineered immune cell according to claim 12, wherein the engineered immune cell is a CAR-T cell.

14. A pharmaceutical composition, wherein the pharmaceutical composition contains the CAR according to any one of claims 1 to 8, the nucleic acid molecule according to claim 9, the vector according to claim 10, the host cell according to claim 11, and/or the engineered immune cell according to claim 12 or 13, and a pharmaceutically acceptable carrier, diluent or excipient.

15. An engineered immune cell according to claim 12 or 13, or a pharmaceutical composition according to claim 14, for use in preventing and/or treating diseases associated with high TSHR expression.
